# EUROPEAN PATENT SPECIFICATION

(11) **EP 1 613 655 B1**
(45) Date of publication and mention of the grant of the patent: **11.08.2010**
(21) Application number: 04727315.6
(22) Date of filing: 14.04.2004
(51) Int. Cl.: C07K 16/12, A61K 39/40, C12Q 1/68, G01N 33/563

(54) **CLOSTRIDIUM DIFFICILE FOCUSSED ANTIBODIES**
GEGEN CLOSTRIDIUM DIFFICILE GERICHTETE ANTIKÖRPER
ANTICORPS SPECIFIQUES A CLOSTRIDIUM DIFFICILE

(30) Priority: 17.04.2003 GB 0309126
(43) Date of publication of application: 11.01.2006
(73) Proprietor: NEUTEC PHARMA PLC, Manchester M13 9WL (GB)
(72) Inventor: BURNIE, James, Peter, Alderley Edge Cheshire SK9 7PY (GB); MATTHEWS, Ruth, Christine, Alderley Edge Cheshire SK9 7PY (GB)
(74) Representative: Arends, William Gerrit
(86) International application number: PCT/GB2004/001619
(87) International publication number: WO 2004/094474

(56) References cited:
- WO-A-00/12562
- WO-A-97/20932
- WO-A-03/052416
- DE-A- 19 739 685
- WHITE HARRY ET AL: "Analysis of immunoglobulin (Ig) isotype diversity and IgM/D memory in the response to phenyl-oxazolone" JOURNAL OF EXPERIMENTAL MEDICINE, vol. 191, no. 12, 19 June 2000 (2000-06-19), pages 2209-2219, XP002293496 ISSN: 0022-1007
- NIE XIAOBO ET AL: "Immunization with immune complex alters the repertoire of antigen-reactive B cells in the germinal centers" EUROPEAN JOURNAL OF IMMUNOLOGY, vol. 27, no. 12, December 1997 (1997-12), pages 3517-3525, XP009035552 ISSN: 0014-2980
- HOLT L J ET AL: "Domain antibodies: proteins for therapy" TRENDS IN BIOTECHNOLOGY, ELSEVIER PUBLICATIONS, CAMBRIDGE, GB, vol. 21, no. 11, November 2003 (2003-11), pages 484-490, XP004467495 ISSN: 0167-7799
- XU J L ET AL: "Diversity in the CDR3 region of V(H) is sufficient for most antibody specificities." IMMUNITY JUL 2000, vol. 13, no. 1, July 2000 (2000-07), pages 37-45, ISSN: 1074-7613
- DATABASE MEDLINE [Online] US NATIONAL LIBRARY OF MEDICINE (NLM), BETHESDA, MD, US; 15 May 1993 (1993-05-15), LEVI M ET AL: "A complementarity-determining region synthetic peptide acts as a miniantibody and neutralizes human immunodeficiency virus type 1 in vitro." Database accession no. NLM7685100 & PROCEEDINGS OF THE NATIONAL ACADEMY OF SCIENCES OF THE UNITED STATES OF AMERICA 15 MAY 1993, vol. 90, no. 10, 15 May 1993 (1993-05-15), pages 4374-4378, ISSN: 0027-8424
- DATABASE MEDLINE [Online] US NATIONAL LIBRARY OF MEDICINE (NLM), BETHESDA, MD, US; January 1998 (1998-01), BOURGEOIS C ET AL: "Prophylactic administration of a complementarity-determining region derived from a neutralizing monoclonal antibody is effective against respiratory syncytial virus infection in BALB/c mice." Database accession no. NLM9420291 & JOURNAL OF VIROLOGY JAN 1998, vol. 72, no. 1, January 1998 (1998-01), pages 807-810, ISSN: 0022-538X
- Roitt, I.: "Essential Immunology" , pages 86-88

## Description

The present invention is concerned with methods of identifying antibodies either specific for and which confer immunity against infection by *Clostridium difficile* or antibodies produced after challenge with a *C. difficile* based vaccine.

*C. difficile* is a gram-positive anaerobic bacterium, and is deemed a significant human pathogen causing a spectrum of diseases ranging from mild diarrhoea to fulminant pseudomembranous colitis (PMC) - collectively referred to as *C. difficile* antibiotic-associated diarrhoea (CDAD). CDAD is a common, iatrogenic, nosocomial disease associated with substantial morbidity and mortality, especially in the elderly. Two factors have been assigned main roles in the pathogenesis of CDAD - the suppression of the resident intestinal flora by the administration of antibiotics, and the production by the bacterium of two high molecular weight toxins, exotoxin A and exotoxin B.

The bacterium is endemic in hospitals, and studies have shown that approximately one third of patients receiving antibiotic treatment in acute-care medical wards were colonised by *C. difficile* while in hospital (Kyne, L., et al., 2002, Clin. Infect. Dis. 34(3), pp346-53, PMTD: 11774082). Of these patients, over half went on to develop CDAD while the remainder were symptomless carriers. CDAD is a major factor in extension of patient hospital stay times, and estimates suggest that the cost of this disease in the US exceeds $1.1 billion per year (Kyne, L., *et al*., Supra). Patients suffering from CDAD respond well to a treatment which includes a discontinuation of the inciting antibiotic and treatment with either of the antibiotics metronidazole and vancomycin. However, the use of e.g. vancomycin is one of last resort since it is associated with several problems. Not only may it cause nephrotoxicity, ototoxicity, bone marrow toxicity and the red man syndrome, but the problem with this treatment regime is that the CDAD often returns after successful treatment of the initial episode, and this reoccurrence represents a serious clinical problem. Additionally, there is evidence that *C*. *difficile* is becoming resistant to metronidazole and partially resistant to vancomycin, demonstrating the need for new alternatives in the treatment of CDAD.

Exotoxins A and B which are produced by pathogenic strains of the bacterium are cytotoxic, enterotoxic and proinflammatory, and are considered to be the main virulence factors of this non-invasive microorganism. However, not all infections with toxigenic strains result in disease, prompting the search for additional virulence factors. Bacterial surface expressed antigens represent candidate virulence factors, and are also considered important since such proteins likely mediate the essential functions such as adhesion to the epithelial layer of the gut in the first step of colonization or interaction with mediators of local immunity. In common with many other bacteria, *C. difficile* expresses a crystalline or paracrystalline surface layer (S-layer) on the outer cell surface. Such S-layers comprise proteins or glycoproteins forming a regularly arranged lattice on the external surface of the bacterium, and have previously been shown to be essential for the virulence of pathogens such as *Aeromanas salmonicida* and *Campylobacter fetus*. In contrast to most bacteria which comprise one S-layer, *C. difficile* is known to comprise two superimposed paracrystalline S-layers, each composed of a glycoprotein subunit which varies slightly in apparent molecular weight among different *C. difficile* strains. Most strains of *C. difficile* express two major S-layer proteins (SLPs), one of 32-38 kDa (low-MW SLP) and a second of 42-48 kDa (high-MW SLP). The low-MW SLP appears to be immunodominant and is the antigen most commonly recognised by patients suffering from CDAD, and is the only antigen recognised in EDTA extracts of bacteria by antisera raised in rabbits against whole *C. difficile* cells (Calabi, E. et al., 2001, Mol. Microbiol., 40(5) p1187-99, PMID: 11401722).

During the course of microbial infection various adaptive strategies are employed by the immune system. One such strategy, and arguably the most important, is the production of an antibody response. Antibodies capable of binding antigens displayed by the infectious agent are produced and bind to and allow killing of the microorganism through complement activation, recruitment of macrophage and through direct interaction with the microbe itself. The therapeutic efficacy of antibodies capable of binding a given antigen varies and this is reflected in the fact that antibody production by the immune system matures during the course of an infection and becomes focussed in the case of a patient successfully fighting off an infection.

The antibody response is elicited by the B cell repertoire where individual B cells each produce structurally diverse antibody molecules. The actual size of this B cell/antibody repertoire is unknown, but it is estimated that the random clonal frequency of reactivity for a given antigen may be as high as 1 in 100,000 in cultured B cells (Nobrega, A., et al., Eur J Immunol. 1998 Apr;28(4):1204-15; PMID: 9565360). During the course of infection, antibodies capable of binding the pathogen are selected for by changes in the B cell population resulting in key antibodies being produced in large numbers. The mechanisms for these changes include clonal expansion, isotype switching, and somatic mutation of immunoglobulin variable regions. B cells responsible for generating antibodies which are able to bind a pathogen multiply, thus skewing the B cell repertoire and changing the proportions of B cells.

Non-antibiotic based therapeutic regimes for the treatment/prevention of *C. difficile* infection are based upon vaccination and passive immunization. Vaccination treatment comprises administering to a patient either a nucleic acid sequence encoding an immunogenic fragment of the *C. difficile* surface layer protein or a variant or homologue thereof, or an equivalent polypeptide fragment (as disclosed in WO 02/062379). Passive immunotherapy is typically achieved by administering to a patient a monoclonal antibody specific to an immunogen produced by a pathogen. In general, passive immunotherapy is particularly effective in treating immunocompromised patients who are unable to respond to vaccination, and to patients who need immediate therapy and cannot wait for vaccination to take effect. In the case of a *C. difficile* infection, passive immunization relies on the administration to a patient of toxin-neutralizing polyclonal immune globulin, (as disclosed in WO 99/2030.4), or antibodies raised against the whole bacterium and the toxins (as disclosed in WO 96/07430).

Thus, as can be seen from the prior art, in order to effect treatment of the patient, an immunogen is first isolated and purified, administered to test animals, and cells producing antibody specific against the immunogen cloned. The range of antibodies produced by the clones can then be tested for their therapeutic efficacy and a single monoclonal antibody selected which is then administered to patients to effect passive immunotherapy.

There are, however, several problems associated with current passive immunotherapy regimes aimed at treating *C. difficile* infections. For example, passive immunotherapy requires that there are survivors of the *C. difficile* infection and patients who have been vaccinated. Each batch of antibody can be different leading to difficulties associated with standardisation and administration of the immunotherapeutic reagent. In addition, the problem of inadvertent administration to a patient of adventitious agents (e.g HIV, HBV, HCV, or as yet unidentified agents) is a real one, and up-to-date screening of any immunotherapeutic reagent is required. Finally, the strain variability exhibited by *C. difficile* means that a given antibody may only be useful against certain strains of the bacterium and not against others.

Obviously the techniques involved are somewhat complex, inconvenient, expensive and time consuming. In general they require that an immunogen is isolated from the infecting pathogen and used to generate antibody. Simply isolating the immunogen can be extremely difficult and time consuming, particularly if it comprises carbohydrate or complex nonlinear epitopes (i.e. epitopes having secondary, tertiary and/or quaternary structural features) which cannot be synthesised *in vitro,* and are impossible to isolate and produce as an antigen for use as e.g. a vaccine. The SLPs of *C. difficile* contain a glycoprotein subunit which varies in molecular weight between species. It may be the case that *C. difficile* epitopes are only produced *in vivo* and are not synthesised *in vitro,* consistent with for example *Neisseria gonorrhoeae* infections (the causal agent of gonorrhea) where specific antigens are only expressed upon infection of a host. Such antigens fall into the general class of cryptoantigens. Furthermore, *C. difficile* may display highly labile antigens which are difficult to work with since during use they simply degrade and the epitope they display is lost. With regard to this range of epitopes/antigens whose identification and/or *in vitro* use is of great difficulty or impossible, the present invention overcomes these disadvantages and provides a solution by providing antibodies whose CDR regions have been generated in response to *C. difficile* epitopes during antibody responses of patients infected with *C. difficile.*

In addition, the prior art typically has to attempt to achieve an equivalent of affinity maturation of antibodies by first synthesising a set of candidate antibodies specific to an antigen, testing them for their binding characteristics and then modifying the sequences of the candidates in order to optimise the binding. A thorough attempt at affinity maturation (i.e. optimising antibody binding) can require the synthesis of thousands of different antibodies, which can be costly and time-consuming. Because the antibodies of the present invention are obtained from patients who have either been infected by a pathogen displaying the antigen or who have been vaccinated with an antigen, they have by their very nature and definition already undergone affinity maturation, as is most clearly demonstrated by the sequences of their CDR3 regions of the variable heavy and variable light chains (i.e. the CDR-H3 and CDR-L3 regions).

DE19739685 discloses monoclonal antibodies capable of recognizing and neutralizing epitopes from the ligand, the tranlocation domain or the catalytic domain of the enterotoxin /toxin A) and cytotoxin (toxin B) from Clostridium difficile.

According to the present invention there is provided a method for identifying candidate sequences of at least the CDR3 region of antibodies specific against at least one antigen produced by *C. difficile* during an infection or against a vaccine, comprising the steps of:
(i) with B cells isolated from at least one patient who has been infected by *Clostridium difficile* or administered said vaccine, sequencing at least the CDR3 region of the VH and/or VL coding regions of said B cells; and
(ii) correlating said sequenced at least the CDR3 regions of the VH and/or VL coding regions of said B cells from said at least one patient to identify a set of candidate sequences for at least a CDR3 region of antibodies specific against said at least one antigen produced by *Clostridium difficile* or against said vaccine, each of said set of candidate CDR3 sequences or a sequence having at least 80% homology therewith occurring in total at a frequency of at least 1 percent in the set of sequences determined at step (i).

Examples of patients used as a source of B cells in such a method are humans and other mammals such as mice, rabbits, rats, baboons, monkeys and apes.

In certain embodiments of the invention, step (i) may comprise the steps of:
(i)
   (a) isolating B cells from at least one patient who has been infected by *Clostridium difficile* or administered said vaccine; and
(i)
   (b) sequencing at least the CDR3 region of the VH and/or VL coding regions of said B cells;

The sequences occurring in total at a frequency of at least 1 percent in the set of sequences determined at step (i) may be the candidate CDR3 sequences or a sequence having at least 80% homology, for example 85, 90, 95, 96, 97, 98 or 99% homology therewith. Sequence homology is as determined using the BLAST2 program (Tatusova TA et al., FEMS Microbiol Lett. 1999 May 15;174(2):247-50; PMID: 10339815) at the National Center for Biotechnology Information, USA (www.ncbi.nlm.nih.gov) with default parameters.

For example, the sequences occurring in total at a frequency of at least 1 percent in the set of sequences determined at step (i) may be the candidate CDR3 sequences or a sequence having 1 or 2 amino acid changes therefrom. In general, the frequency of a dominant sequence is added together with the frequency of any sequence showing >80% homology, such that any sequence exhibiting a frequency of 1 % or greater is then deemed a candidate sequence. For example, a first sequence may occur at a frequency of 0.7 percent, and first, second, third and fourth sequences each having a single amino acid change there from each occur at a frequency of 0.1 % - the total occurrence is therefore 1.1 % and the dominant antibody sequence (occurring at a frequency of 0.7%) is therefore a candidate CDR3 sequence.

This is fundamentally different to prior art methods of making a library of antibodies in a vector such as a phage library where antibodies are isolated by panning and which require binding with a specific (known) antigen in a structurally- and charge-specific state. An analogy by which the two can be compared would be to say that the prior art making of a library and subsequent panning is like going into a library, picking up a book and hoping that it is the right one. In comparison, the present invention is like going into a library, reading all of the books in the library and finding that one book is dominant - that there are many copies of it - and that it is relevant to the disease to be treated.

In addition, the prior art use of library systems has a number of significant problems - in particular, some antibodies produced by a library may cause the death of the organism expressing them and therefore they simply cannot be detected. This is not a particular problem when e.g. looking for antibodies specific to cancers, but when one is searching for antibodies specific to an antigen from a pathogen which might be homologous to one produced by the host expression system (e.g. *Escherichia coli*) then important antibodies cannot be expressed. The use of e.g. *E. coli* to express libraries of e.g. human antibodies also suffers from the problem of codon usage - codons used by humans for specific amino acids can frequently not be the optimum ones for the same amino acid in *E. coli* or other host systems. This means that an important antibody might not be expressed (or at least not in sufficient quantities) since the codons in its sequence are highly inefficient in *E. coli*, resulting in the *E. coli* being unable to read through and express it in full. Codon optimisation of antibody libraries is obviously not an option since the libraries would first have to be sequenced, which defeats the main advantages of using libraries. Since the present invention sequences antibodies directly from a patient, it avoids this problem. The correlation step (ii) may also correlate the occurrence of the candidate sequences against their occurrence in patients who have not been infected with *Clostridium difficile* or administered the vaccine, sequences only being determined to be candidate sequences if they or a sequence having 80% homology to them occur in total at a frequency of less than 1 percent in the non-infected/vaccinated patients.

The at least one antigen produced by the micro-organism may of course be an immunogen.

The present invention provides unique opportunities for understanding antibody responses to infection which were not previously available, and provides novel diagnostic and therapeutic opportunities.

In particular, since the methods of the present invention bypass the need to isolate an antigen from *C. difficile,* the difficulties associated with identifying e.g. complex nonlinear epitopes, epitopes containing carbohydrate, or cryptoantigens are avoided. Instead the methods facilitate the determination of the identity of antibodies specific against one or more antigens produced by the bacterium. As is explained below, the methods of the present invention allow the identification of therapeutically effective antibodies and can allow the identification of the most important parts of antibody sequences. This is particularly evident when the B cells of a given patient are sampled at different time points during the course of an infection by *C. difficile -* as the patient's immune system becomes focussed on producing therapeutically effective antibodies against the bacterium, a focussing of antibody sequences is observed, and the development of variable and non-variable regions within the CDR parts of the VH and/or VL sequences is observed. In addition, the methods of the present invention can allow the identification of antibodies best suited to the treatment of *C. difficile* infection in particular groups of patients, such as age, sex and racial groups. Similarly by comparing the antibody sequences of patients who have recovered from infection with *C. difficile* with those that have not recovered from infection, ineffective antibody sequences (which might be produced by both sets of patients) can be identified.

The B cells isolated from the at least one patient can be peripheral B-cell lymphocytes (PBLs), and can be isolated from a blood sample from the at least one patient. B-cells can also be isolated from other sources and the present invention extends to their use. For example, B-cells can be isolated from spleen.

Antibody CDR sequences are determined as detailed in the "Experiments" section using standard techniques and definitions of their start and end.

As is detailed below, the antibody sequences are determined from patients with *C. difficile* infections. These sequences can be derived from B cell immunoglobulin mRNA and hence reflect expressed antibodies and repertoires therein. The sequences determined according to the present invention need not be the sequences of whole antibody molecules - they comprise at least the VH and/or VL sequences which specify the regions responsible for antigen binding.

From the nucleotide sequences determined by the initial sequencing, putative amino acid sequences for the VH and/or VL regions can be determined using standard algorithms and software packages (e.g. see www.mrc-lmb.cam.ac.uk/pubseq/, the Staden package and Gap4 programs; Rodger Staden, David P. Judge and James K. Bonfield. Managing Sequencing Projects in the GAP4 Environment. Introduction to Bioinformatics. A Theoretical and Practical Approach. Eds. Stephen A. Krawetz and David D. Womble. Human Press Inc., Totawa, NJ 07512 (2003); Rodger Staden, David P. Judge and James K. Bonfield. Analysing Sequences Using the Staden Package and EMBOSS. Introduction to Bioinformatics. A Theoretical and Practical Approach. Eds. Stephen A. Krawetz and David D. Womble. Human Press Inc., Totawa, NJ 07512 (2003)). These can be further characterised to determine the CDR (Complementarity Determining Region) parts of the VH and/or VL sequences, particularly CDR1, CDR2 and CDR3. Methods for determining the putative amino acid sequences and identifying CDR regions are well known and detailed below.

As well as the VH and/or VL amino acid sequences, several pieces of additional information can be used in the correlation step:
(i) the strain of *C. difficile* causing the patient's infection;
(ii) the time point during the infection process at which antibody sequences were sampled
(iii) patient details - none or more of: sex, race, and age; and
(iv) the range of complementarity determining regions (CDR) ie the variable regions of the antibody that undergo direct antigen binding/contact.

Thus in a preferred method according to the present invention, B cells can be used which have been isolated from the at least one patient at a plurality of time points during infection of the at least one patient by *C. difficile* (or post-vaccination), correlation step (ii) correlating the time point during infection of the at least one patient by *C. difficile* at which the B cells are isolated.

Similarly, in a preferred method according to the present invention, B cells can be used which have been isolated from at least two patients, at least one of whom has recovered from infection by *C. difficile,* and at least one of whom has not recovered from infection by *C. difficile,* correlation step (ii) correlating the recovery of the at least two patients from infection by *C. difficile.*

Similarly, in a preferred method according to the present invention, B cells can be used which have been isolated from at least two patients, the patients being infected by strains of *C*. *difficile* producing the at least one antigen, correlation step (ii) correlating the sequenced at least the VH and/or VL coding regions of the B cells to identify a set of candidate sequences for antibodies, each of which is specific against at least one shared antigen produced by the different strains of *C. difficile* or is specific against different antigens produced by the different strains of *C*. *difficile.*

The sequencing of the VH and/or VL regions can also be used to identify the surrounding antibody framework, and this can be used to determine the antibody isotype. This can then be used in the correlation step to determine whether specific antibody isotypes are particularly useful.

By the term "therapy" is meant any treatment which is designed to cure, alleviate, remove or lessen the symptoms of, or prevent or reduce the possibility of contracting any disorder or malfunction of the human or animal body

The invention is suitable for identifying antibody sequence useful in treating *C. difficile* infection.

Using the abovementioned additional information fields in the correlation step (ii), it is easy to perform the correlation using e.g. only CDR sequences, CDRs from either a given patient or a given infection or both, CDRs from a given site or patient group, or CDRs from different sampling times over the course of infection.

These correlations are CDR-specific because these are the hypervariable regions responsible for antigen binding and sequence diversity of the antibody repertoire (although the framework regions can be used in the correlation step as well to reveal the antibody isotype).

The present invention is useful in a wide range of applications, e.g. antibody therapy, and vaccine studies.

### Antibody Therapy:

This involves the passive transfer of antibody to non-immune individuals (e.g. patients undergoing chemo-/radio-therapy, immunosuppression for organ transplantation, immunocompromised due to underlying conditions such as diabetes, trauma etc, also the very young or very old).

The present invention can be used to determine the sequences of antibodies conferring immunity by looking for over-represented VH and/or VL sequences in patients who have overcome infection. These protective antibodies can be re-synthesised at the genetic level, over-expressed in *E. coli* (or other expression systems) and purified. The resultant purified recombinant antibody can then be administered to patients as a passive immunotherapy. Antibodies can also be ordered from commercial suppliers such as Operon Technologies Inc., USA (www.operon.com) by simply supplying them with the sequence of the antibody to be manufactured.

Therapeutically useful sequences can be identified in a number of ways which can be used in the correlation step (ii):
1) By looking for antibody sequence over-representation in patients who have recovered from *C. difficile* infection - B cells that produce antibodies capable of pathogen binding undergo clonal expansion, hence high frequency antibody sequences are most likely to bind pathogen and confer immunity.
2) By following alterations in the antibody repertoire over the course of *C. difficile* infection. During infection, antibodies undergo a maturation process to improve pathogen binding and this is characterised by sequence alterations. Also, B cell clonal expansion is more prevalent in the final stages of infection where the infection is cleared. The most frequent antibodies in the repertoire are chosen as candidates for immunotherapy. Following the maturation process the candidate antibody will demonstrate which key amino acid residue alterations improve antigen binding and this information can be used to improve antibody design.
3) Analysis of the repertoires from different patients with *C. difficile* infection can identify shared and identical protective antibodies. These antibodies are attractive choices for immunotherapy as their occurrence in different patients suggests a strong positive selection in their favour; hence an important role in antibody-based immunity.
4) Analysis of the repertoires from patients infected with different strains of *C. difficile*. In this situation, if a common antibody sequence is found in the repertoires for both strains, the antibody may be useful in treating both strains of the bacterium, ie displaying a broad spectrum.
5) Analysis of the repertoires for affinity maturation of sequences.

### Vaccination Studies:

Vaccination protects against infection by priming the immune system with pathogen-derived antigen(s). Vaccination is effected by a single or repeated exposures to the pathogen-derived antigen(s) and allows antibody maturation and B cell clonal expansion without the deleterious effects of the full-blown infectious process. T cell involvement is also of great importance in effecting vaccination of patients. The present invention can be used to monitor the immunisation process with experimental *C. difficile* vaccines. Subjects are given the experimental vaccine and VH and/or VL sequences are amplified from the patient and the antibody repertoire analysed as described above. Qualitative and quantitative assessment of the vaccination process is possible:
1) The VH/VL repertoire of a group of patients who have been administered an experimental *C. difficile* vaccine is assessed. A precise molecular dissection of the resulting antibody response to the vaccine can be performed to determine (a) clonal expansion of protective antibodies, (b) protective antibody production in different populations (for example differing ethnic groups with different genetic backgrounds, as well as e.g. age and sex groups), and (c) the long term effect of the vaccine i.e. the antibody response over the long term - long term antibody memory in the immune system as well as any autoimmune defects;
2) Where vaccination results in an increased frequency of a given antibody, the antibody sequence can easily be cloned and expressed and used in animal models of infection. If the antibody is protective, it may be useful in itself as an immunotherapy, but this also demonstrates that the vaccine is likely to be protective without subjecting the human subject to experimental infection.

Also disclosed herein is a method of manufacture of a medicament for the treatment of a *C. difficile* infection which produces at least one antigen, comprising the steps of:
(i) performing a method according to the present invention to identify a set of candidate sequences for antibodies specific against the at least one antigen produced by *C. difficile*; and
(ii) synthesising at least one antibody comprising a said candidate sequence specific against the at least one antigen produced by *C. difficile.*

Medicaments and methods of treatment disclosed herein will be readily apparent to one skilled in the art. Medicaments may be prepared using pharmaceutically acceptable carriers, diluents or excipients (Remington's: The Science and Practice of Pharmacy (1995)Mack Publishing Company, Easton, PA, USA). The medicaments and methods of treatment may be effected using a pharmaceutically effective amount of the antibody/antigen-binding fragment. Appropriate dosages will be readily apparent to one skilled in the art and may be readily determined, for example by means of dose-response experiments

Also disclosed herein is a method of treatment of an infection of a patient by *C. difficile* which produces at least one antigen, comprising the steps of:
(i) performing a method according to the present invention to identify a set of candidate sequences for antibodies specific against the at least one antigen produced by *C. difficile*;
(ii) synthesising at least one antibody comprising a said candidate sequence specific against the at least one antigen produced by *C. diffcile*; and
(iii) administering a therapeutically effective quantity of said at least one synthesised antibody to said patient.

Also provided according to the present invention is a method of producing a database which identifies candidate sequences for antibodies specific against at least one antigen produced by *C. difficile,* comprising the steps of:
(i) performing a method according to the present invention to identify a set of candidate sequences for antibodies specific against the at least one antigen produced by *C. difficile*; and
(ii) storing the data produced by said method in said database.

Preferably the method comprises the step of:
(iii) producing a report comprising the data produced by said method.

Also disclosed herein is a method for determining the efficacy of a vaccine, comprising the steps of:
(i) with B cells isolated from at least one patient who has been administered said vaccine, sequencing at least the CDR3 region of the VH and/or VL coding regions of said B cells; and
(ii) correlating said sequenced at least the CDR3 region of the VH and/or VL coding regions of said B cells to identify a set of candidate sequences for at least the CDR3 region of antibodies specific against said vaccine, each of said set of CDR3 candidate sequences or a sequence having at least 80% homology therewith occurring in total at a frequency of at least 1 percent in the set of sequences determined at step (i).

Step (i) above may comprise the steps of:
(i)(a) administering said vaccine to at least one patient;
(i)(b) isolating B cells from said at least one patient; and
(i)(c) sequencing at least the CDR3 region of the VH and/or VL coding regions of said B cells.

Correlation step (ii) may comprise correlating said sequenced at least a CDR3 region of the VH and/or VL coding regions of said B cells with sequenced at least a CDR3 region of the VH and/or VL coding regions of B cells isolated from at least one patient who has been infected with *C. difficile* against which vaccination with said vaccine is intended to stimulate a protective immune response.

As described above, additional information may be used in the correlation of step (ii), including:
(i) the time since administration of the vaccine to the patient;
(ii) patient details - none or more of: sex, race, and age; and
(iii) the range of complementarity determining regions (CDR) ie the variable regions of the antibody that undergo direct antigen binding/contact.

The antibody sequences determined from patients who have been administered the vaccine can be compared with antibody sequences isolated from patients who have been infected with *C. difficile* against which the vaccine is intended to stimulate a protective immune response in patients. Thus it is possible to determine whether the vaccine results in the generation of antibodies which are also produced in response to infection by *C. difficile* itself. In particular, the comparison can be made using antibody sequences determined from patients who have recovered from infection by *C. difficile.*

In particular the method may determine the efficacy of the vaccine in stimulating a protective immune response against *C. difficile* against which vaccination with the vaccine is intended to stimulate a protective immune response.

Also disclosed is a method of producing a database which identifies the efficacy of a vaccine, comprising the steps of:
(i) performing a method described above to determine the efficacy of said vaccine; and
(ii) storing the data produced by said method in said database.

Also disclosed is a method of generating a report which identifies the efficacy of a vaccine, comprising the steps of:
(i) performing a method described above to determine the efficacy of said vaccine; and
(ii) producing a report comprising the data produced by said method.

Also disclosed is a diagnostic test method for identifying a *Clostridium difficile* infection in a patient, comprising the steps of:
(i) with B cells isolated from said patient, sequencing at least the CDR3 region of the VH and/or VL coding regions of said B cells;
(ii) comparing said sequenced at least said CDR3 region of the VH and/or VL coding regions of said B cells with a set of sequences for at least the CDR3 region of antibodies specific against *Clostridium difficile,* and determining whether each of said set of CDR3 sequences or a sequence having at least 80% homology therewith occurs in total at a frequency of at least 1 percent in the set of sequences determined at step (i); and
(iii) correlating the results of comparison step (ii) to determine the presence or absence of a *Clostridium difficile* infection in said patient.

Also disclosed is a diagnostic test method for determining the susceptibility of a patient to *Clostridium difficile* infection, comprising the steps of:
(i) with B cells isolated from said patient, sequencing at least the CDR3 region of the VH and/or VL coding regions of said B cells;
(ii) comparing said sequenced at least said CDR3 region of the VH and/or VL coding regions of said B cells with a set of sequences for at least the CDR3 region of antibodies specific against *Clostridium difficile,* and determining whether each of said set of CDR3 sequences or a sequence having at least 80% homology therewith occurs in total at a frequency of at least 1 percent in the set of sequences determined at step (i); and
(iii) correlating the results of comparison step (ii) to determine the susceptibility of said patient to *Clostridium difficile* infection.

The diagnostic test method could be used to sequence the CDR3 sequences of patients in for example a hospital ward, to determine which patients were susceptible to infection by the bacterium. The presence of *C. difficile* specific antibodies in a sample (as determined by sequencing of CDR3 regions) would likely indicate that such patients were capable of mounting a protective immune response toward the bacterium and would not require antibiotic treatment. In contrast, those patients identified who had not produced *C. difficile* specific antibodies could be identified as being immunocompromised and therefore candidates for antibiotic treatment during the course of a *C. difficile* infection.

Also disclosed is a diagnostic test kit for performing a diagnostic test method described above. The kit may contain reagents (e.g. PCR primers) which would enable a person skilled in the art to identify CDR3 sequences from a patient.

As regards antibodies disclosed herein, particularly synthetic antibodies (the term "antibody" is also considered to incorporate antigen binding fragments of antibodies unless the context otherwise requires), the antibody may be a whole antibody or an antigen binding fragment thereof and may in general belong to any immunoglobulin class. Thus, for example, it may be an IgM, IgG or an IgA antibody. The antibody or fragment may be of animal, for example, mammalian origin and may be for example of murine, rat, sheep or human origin. It may be a natural antibody or a fragment thereof, or, if desired, a recombinant antibody fragment, i.e. an antibody or antibody fragment which has been produced using recombinant DNA techniques.

Particular recombinant antibodies or antibody fragments include, (1) those having an antigen binding site at least part of which is derived from a different antibody, for example those in which the hypervariable or complementarity determining regions of one antibody have been grafted into the variable framework regions of a second, different antibody (as described in, for example, EP 239400); (2) recombinant antibodies or fragments wherein non-Fv sequences have been substituted by non-Fv sequences from other, different antibodies (as described in, for example, EP 171496, EP 173494 and EP 194276); or (3) recombinant antibodies or fragments possessing substantially the structure of a natural immunoglobulin but wherein the hinge region has a different number of cysteine residues from that found in the natural immunoglobulin but wherein one or more cysteine residues in a surface pocket of the recombinant antibody or fragment is in the place of another amino acid residue present in the natural immunoglobulin (as described in, for example, WO 89/01782 and WO 89/01974).

Teachings of texts such as Harlow, E. and Lane, D. ("Using Antibodies: A Laboratory Manual", Cold Spring Harbor Laboratory Press, New York, 1998) further details antibodies, antibody fragments, their preparation and use.

The antibody or antibody fragment may be ofpolyclonal or monoclonal origin. It may be specific for at least one epitope.

Antigen binding antibody fragments include, for example, fragments derived by proteolytic cleavage of a whole antibody, such as F(ab')2, Fab' or Fab fragments, or fragments obtained by recombinant DNA techniques, for example Fv fragments (as described, for example, in WO 89/02465).

Where it is desired to produce recombinant antibodies, these may be produced using, for example, the methods described in EP 171469, EP 173494, EP 194276 and EP 239400. Antibody fragments may be produced using conventional techniques, for example, by enzymatic digestion with pepsin or papain.

Antibodies may be labelled with a detectable label or may be conjugated with an effector molecule, for example a drug eg. an antibacterial agent or a toxin or an enzyme, using conventional procedures.

Where "PMID:" reference numbers are given for publications, these are the PubMed identification numbers allocated to them by the US National Library of Medicine, from which full bibliographic information and abstract for each publication is available at www.ncbi.nlm.nih.gov.

The invention will be further apparent from the following description.

There are disclosed herein the general principles for the isolation and DNA sequencing of VH and/or VL antibody gene fragments from B cells. There is disclosure of cloning into a DNA sequencing vector using the T-tailing principle - random orientation, and the determining of the nucleotide sequence of forward and reverse strands using M13 forward and reverse primers.

There is also disclosure of resynthesised recombinant antibody gene cassette. VH and VL regions are linked with a glycine serine-rich linker. Each variable domain contains three Complementarity Determining Regions (CDRs) which participate in antigen binding.

### EXPERIMENTS

The experiments below detail methods for identifying antibody VH and/or VL CDR sequences that confer immunity in patients with *C*. *difficile* infection. These sequences can then be used to produce synthetic antibodies and these synthetic antibodies are suitable for administration to patients for therapy. Also detailed are the VH and VL CDR3 sequences identified by the methods.

Also detailed are methods of determining the efficacy of a vaccine in generating a desired immune response in a patient.

Unless stated otherwise, all procedures were performed using standard protocols and following manufacturer's instructions where applicable. Standard protocols for various techniques including PCR, molecular cloning, manipulation and sequencing, the manufacture of antibodies, epitope mapping and mimotope design, cell culturing and phage display, are described in texts such as McPherson, M.J. et al. (1991, PCR: A practical approach, Oxford University Press, Oxford), Sambrook, J. et al. (1989, Molecular cloning: a laboratory manual, Cold Spring Harbour Laboratory, New York), Huynh and Davies (1985, "DNA Cloning Vol I - A Practical Approach", IRL Press, Oxford, Ed. D.M. Glover), Sanger, F. et al. (1977, PNAS USA 74(12): 5463-5467), Harlow, E. and Lane, D. ("Using Antibodies: A Laboratory Manual", Cold Spring Harbor Laboratory Press, New York, 1998), Jung, G. and Beck-Sickinger, A.G. (1992, Angew. Chem. Int. Ed. Eng., 31: 367-486), Harris, M.A. and Rae, I.F. ("General Techniques of Cell Culture", 1997, Cambridge University Press, ISBN 0521 573645), "Phage Display of Peptides and Proteins: A Laboratory Manual" (Eds. Kay, B.K., Winter, J., and McCafferty, J., Academic Press Inc., 1996, ISBN 0-12-402380-0).

Reagents and equipment useful in, amongst others, the methods detailed herein are available from the likes of Amersham (www.amersham.co.uk), Boehringer Mannheim (www.boehringer-ingeltheim.com), Clontech (www.clontech.com), Genosys (www.genosys.com), Millipore (www.millipore.com), Novagen (www.novagen.com), Perkin Elmer (www.perkinelmer.com), Pharmacia (www.pharmacia.com), Promega (www.promega.com), Qiagen (www.qiagen.com), Sigma (www.sigma-aldrich.com) and Stratagene (www.stratagene.com).

The term "antibody" in its various grammatical forms is used herein to refer to immunoglobulin molecules and immunologically active portions of immunoglobulin molecules, i.e., molecules that contain an antibody combining site or paratope. Such molecules are also referred to as "antigen binding fragments" of immunoglobulin molecules.

Illustrative antibody molecules are intact immunoglobulin molecules, substantially intact immunoglobulin molecules and those portions of an immunoglobulin molecule that contain the paratope, including those portions known in the art as Fab, Fab', F(ab')2, scFv and F(v).

The term "antibody combining site" refers to that structural portion of an antibody molecule comprised of a heavy and light chain variable and hypervariable regions that specifically binds (immunoreacts with) antigen.

The identification and sequencing of B cells producing antibodies and the analysis of the resulting data comprises the following basic steps:
(1) Isolation of VH and/or VL coding regions from circulatory B cells of human patients.
(2) Determining the Nucleotide sequence of VH and/or VL repertoires.
(3) Determination of VH and/or VL primary amino acid sequences.
(4) Extraction of CDR regions *in silico* - incorporation into the database
(5) Detection of dominant CDR & framework regions in VH and/or VL repertoire.
(6) Construction & Production of therapeutic recombinant antibodies from dominant antibody sequences.

### 1. Isolation of VH and/or VL coding regions from circulatory B cells of human patients

For this method, human patients with *C. difficile* infection were selected as donors of immunised B cells. The criteria for selection were:
(a) The patient must exhibit a pronounced antibody response to *C. difficile,* detectable for example by Western blotting. Samples of antibodies were collected from a patient blood sample. Samples were diluted and tested for immunoreactivity by Western blotting using antigen(s) derived from *C. difficile.* In such an assay, patients exhibiting strong antibody responses showed pronounced recognition/antibody binding of *C. difficile* antigens as detected using anti-human polyclonal detection antibodies.
(b) The patient has survived the course of *C. difficile* infection - this improves the chance of generating B cell responses for antibodies capable of neutralizing the bacterium.

Peripheral B-cell lymphocytes (PBLs) were collected from infected patient blood samples. For this heparinised blood was diluted in PBS (20 ml total) and overlaid onto a 15 ml cushion of Ficol Hypaque (Pharmacia; unless stated otherwise, all chemicals and culture media were purchased from Sigma, UK) in a 30 ml centrifuge tube. The PBLs were then collected by centrifugation (400 x g, 5 minutes) and washed in PBS and harvested by centrifugation again. RNA was prepared from PBLs using the QuickPrep mRNA purification kit (Pharmacia) exactly according to the manufacturers instructions. The isolated mRNA was used to prepare cDNA via a reverse transcriptase reaction (Promega cDNA synthesis kit). For this 2µg of mRNA was re-suspended in 16 µL nuclease-free water and heated to 65 °C for 3 minutes (to denature secondary structure) and then immediately chilled on ice for 1 minute. The mRNA was then added to the following cocktail: 8 µL 25 mM MgCl₂, 4 µL dNTP mix (10 mM with respect to each ribonucleotide triphosphate), 1 µL RNAsin 40 u µL⁻¹ stock solution, 1.2 µL AMV reverse transcriptase (25 u µL⁻¹ stock solution), 6 µL cDNA 10 pmol µL primer. The mixture was incubated at 42 °C for 1 hour and then incubated at 100 ° C for 3 minutes to stop the reaction.

DNA coding for antigen binding regions was then amplified by PCR using the cDNA prepared from patients PBLs. For this, the cDNA was used in the following PCR reaction to produce either heavy chain or light chain-derived antibody variable region DNA (see Figure 1): 2.5 µL cDNA, 33 µL water, 4 µL dNTP mix (25 mM with respect to each deoxynucleotide triphosphate), 5 µL Taq reaction buffer (Perkin Elmer), 2.5 µL of an equimolar primer mix (final concentration of 20 pmol with respect to each primer) and 0.5 µL Taq DNA polymerase ( 1 u µL⁻¹, Perkin Elmer Corp). The forward and reverse primers used in these reactions are depicted in Figure 1, and their respective nucleotide sequences are listed in Table 1. PCR reaction conditions were 94 °C for 1 minute, 57 °C for 1 minute and 72 °C for 2 minutes for a total of 30 cycles, with an extended denaturation (94 °C for 5 minutes) prior to cycle 1 and an additional extension step after the end of cycle 30 (72 °C for 10 minutes). PCR was performed using a Perkin Elmer 9700 GeneAmp PCR machine. 5 µL of the PCR reaction was run on a 1% agarose gel to check the amplification of the expected 393 base pair (bp) product. The remaining product was run on a 0.8% low melting point (LMP) agarose gel and the 393 bp band was excised using a clean scalpel blade. DNA was extracted from the agarose gel slice using a GeneClean II Kit (Anachem, Luton, UK).

The PCR resulted in two fragment types -derived from VH and VL regions respectively, depending on the PCR primer sets used (for details of primers sets for antibody genes see Table 1).

Table 1: Primers used in generating and sequencing VH and VL gene fragments. All sequences are given in the 5' to 3' direction. Use of primers is described above.

**Table 1**

| Primers used in cDNA synthesis reactions | | SEQ ID NO: |
|---|---|---|
| HuIgG1-4FOR | GTC CAC CTT GGT GTT GCT GGG CTT | 01 |
| HuCLFOR | AGA CTC TCC CCT GTT GAA GCT CTT | 02 |
| | | |

| Primers for primary PCR | | SEQ ID NO: |
|---|---|---|
| HuJH1-2FOR | TGA GGA GAC GGT GAC CAG GGT GCC | 03 |
| HuJH3FOR | TGA AGA GAC GGT GAC CAT TGT CCC | 04 |
| HuJH4-5FOR | TGA GGA GAC GGT GAC CAG GGT TCC | 05 |
| HuJH6FOR | TGA GGA GAC GGT GAC CGT GGT CCC | 06 |
| HuVH1aBACK | CAG GTG CAG CTG GTG CAG TCT GG | 07 |
| HuVH2bBACK | CAG GTG AAC TTA AGG GAG TCT GG | 08 |
| HuVH3aBACK | CAG GTG CAG CTG GTG GAG TCT GG | 09 |
| HuVH4aBACK | CAG GTG CAG CTG CAG GAG TCG GG | 10 |
| HuVH4bBACK | CAG GTG CAG CTA CAG CAG TGG GG | 11 |
| HuVH5aBACK | GAG GTG CAG CTG TTG CAG TCT GC | 12 |
| HuVH6aBACK | CAG GTA CAG CTG CAG CAG TCA GG | 13 |
| HuJK1FOR | ACG TTT GAT TTC CAC CTT GGT CCC | 14 |
| HuJK2FOR | ACG TTT GAT CTC CAG CTT GGT CCC | 15 |
| HuJK3FOR | ACG TTT GAT ATC CAC TTT GGT CCC | 16 |
| HuJK4FOR | ACG TTT GAT CTC CAC CTT GGT CCC | 17 |
| HuJK5FOR | ACG TTT ATT CTC CAG TCG TGT CCC | 18 |
| HuVK1BACK | GAC ATC CAG ATG ACC CAG TCT CC | 19 |
| HuVK2BACK | GAT GTT GTG ATG ACT CAG TCT CC | 20 |
| HuVK3BACK | GAA ATT GTG TTG ACG CAG TCT CC | 21 |
| HuVK4BACK | GAC ATC GTG ATG ACC CAG TCT CC | 22 |
| HuVK5BACK | GAA ACT ACA CTC ACG CAG TCT CC | 23 |
| HuVK6BACK | GAA ATT GTG CTG ACT CAG TCT CC | 24 |

| Primers for DNA Sequencing | | |
|---|---|---|
| M13forward | GTA AAA CGA CGG CCA GT | 25 |
| M13reverse | AAC AGC TAT GAC CAT G | 26 |

VH and/or VL gene fragments were cloned into cloning vector pGEM-T easy (Promega Corporation) to facilitate DNA sequencing. For this 3 µg PCR product was prepared for restriction using QIAquick PCR purification spin columns (Qiagen, UK) according to the manufacturers instructions. DNA was eluted from the spin column in 40 µL buffer EB. Purified PCR product (2 µL) was mixed with 1 µL pGEM-T easy vector, 6 µL water and 1 µL DNA ligase and the mixture ligated for 1 h at room temperature. Ligations were then transformed into electrocompetent *E. coli* TG1 cells (Stratagene) by electroporation, and plated out onto agar plates containing Ampicillin 100 µg ml⁻¹ IPTG (100 µM) and X-gal (0.006 % w/v). Colonies were allowed to grow overnight at 37 °C and then stored at 4 °C. Recombinant colonies are identified as white colonies on this media.

### 2. Determining the nucleotide sequence of VH and/or VL repertoires

DNA was first prepared from VH and/or VL recombinant *E. coli* clones. For this, individual colonies were each transferred to 1.2 ml LB broth supplemented with Ampicillin 100 µg ml⁻¹ using a 96 well plate format. Cultures were the grown at 37 °C for 24 hours. Bacterial cells were harvested by centrifugation at 4,000 x g, 30 minutes and the supernatants discarded. Plasmid DNA was prepared using Wizard SV 96 plasmid purification kits (Promega Corporation) essentially following the manufacturer's instructions. Yields of plasmid DNA were typically in the order of 5 µg per 1.2 ml starter culture.

DNA sequencing reactions were performed using the DYEnamic ET dye terminator cycle sequencing kit (Amersham Pharmacia Biotech). Purified plasmid DNA (0.5 µg) was mixed with 8 µL DYEnamic ET terminator reagent premix and 1 µL M13 forward or reverse primer (5 µM) in a total reaction volume of 20 µL. Thermal cycling was then performed using a GeneAmp PCR system 9700 (Perkin Elmer) with the following parameters: 95 °C, 20 seconds; 50 °C, 15 seconds; 60 °C, 1 minute; 30 cycles). Reactions were performed using 96 well format non-skirted ELISA plates (AB Gene). Unincorporated dye terminator were removed using precipitation. For this, ethanol samples were mixed with 2 µL 7.5 M ammonium acetate and 55 µL of 100 % ethanol and centrifuged at 3000 g for 30 minutes. DNA pellets were washed with 70 % ethanol and re-suspended in 20 µL loading solution. Reactions were sequenced using a MegaBACE 1000 DNA sequencer (Amersham Pharmacia Biotech) following the manufacturers instructions (2 kV injection voltage for 30 s with electrophoresis at 6 kV for 200 minutes). Chromatograms were exported using the .scf file format for finishing and archiving.

### 3. Determination of VH and/or VL primary amino acid sequences

DNA sequences of VH and/or VL were determined in both forward and reverse strands (using M13 forward and reverse primers respectively) and compared in order to highlight discrepancies and maintain a high degree of accuracy. This was done using the Staden suite of programs (Staden, R. (1996) The Staden Sequence Analysis Package. Molecular Biotechnology 5, 233-241). First, sequence .scf files were entered into the PREGAP program where vector sequence was stripped off and the quality of the sequence was assessed. Poor quality sequences where the DNA sequence was ambiguous were rejected and re-sequenced. Forward and reverse strands were matched using the GAP program to highlight and resolve areas containing sequencing artefacts. The orientation of the VH and/or VL sequence was noted and reverse complemented where necessary to produce only forward reading frame orientations for translation. VH and VL gene sequences were then translated into amino acid sequences. Each sequence was given a unique identifier (name).

### 4. Extraction of CDR regions in silico - incorporation into the database

A general teaching of identifying CDR regions is at www.bioinf.org.uk/abs/

The following set of rules will allow the definition of the CDRs in an antibody sequence. There are rare examples where these virtually constant features do not occur (for example the human heavy chain sequence EU does not have Trp-Gly after CDR-H3). The Cys residues are the best-conserved feature.

### CDR-L1

| | |
|---|---|
| Start | Approx. residue 24 |
| Residue before | always a Cys |
| Residue after | always a Trp. Typically Trp-Tyr-Gln, but also, Trp-Leu-Gln, Trp-Phe-Gln, Trp-Tyr-Leu |
| Length | 10 to 17 residues |

### CDR-L2

| | |
|---|---|
| Start | always 16 residues after the end of CDR-L1 |
| Residues before | generally Ile-Tyr, but also, Val-Tyr, Ile-Lys, Ile-Phe |
| Length | always 7 residues |

### CDR-L3

| | |
|---|---|
| Start | always 33 residues after end of CDR-L2 |
| Residue before | always Cys |
| Residues after | always Phe-Gly-Xaa-Gly (SEQ ID NO: 92) |
| Length | 7 to 11 residues |

### CDR-H1

| | |
|---|---|
| Start | Approx. residue 26 (always 4 after a Cys) |
| Residues before | always Cys-Xaa-Xaa-Xaa (SEQ ID NO: 93) |
| Residues after | always a Trp. Typically Trp-Val, but also, Trp-Ile, Trp-Ala |
| Length | 10 to 12 residues |

### CDR-H2

| | |
|---|---|
| Start | always 15 residues after the end of CDR-H1 |
| Residues before Residues after | typically Leu-Glu-Trp-Ile-Gly (SEQ ID NO: 94), but a number of variations Lys/Arg-Leu/Ile/Val/Phe/Thr/Ala-Thr/Ser/Ile/Ala |
| Length | 16 to 19 residues; |

### CDR-H3

| | |
|---|---|
| Start | always 33 residues after end of CDR-H2 (always 2 after a Cys) |
| Residues before | always Cys-Xaa-Xaa (typically Cys-Ala-Arg) |
| Residues after | always Trp-Gly-Xaa-Gly (SEQ ID NO: 95) |
| Length | 3 to 25 residues |

### 5. Detection of dominant CDR & framework regions in VH and/or VL repertoire

The database was constructed using SQL Server Database software (Microsoft). This allowed the database to be queried using SQL and allowed CDR1, CDR2 and CDR3 sequences to be extracted from any range of database VH and/or VL sequences in FASTA format. Extracted CDRs were then subject to multiple alignment using CLUSTAL X in such a way so that identical or very similar CDRs are grouped together in blocks (Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680). From this the frequency of recurring CDRs was determined and CDR-dominance in the immunoglobulin repertoire was established. Alternatively, a graphical interface was employed using Probiosys (proBionic EMEA, Amsterdam). ProBiosys prepares dendrograms from CLUSTAL .phy files and allows a rapid visual interpretation of the relationships among large numbers of aligned sequences. The whole process was performed for CDRs from the same patient sampled at different points during an illness, for CDRs from patients with different infections, or from sex-matched, age-matched or race-matched patient groups, depending on the search and the additional information added to the database entries.

### 6. Construction & production of therapeutic recombinant antibodies from dominant antibody sequences

Once dominant antibody sequences have been recognised in a given repertoire, the information can be used to infer the presence of CDRs and frameworks that confer immunity. Selected VH and/or VL sequences can be identified and their gene sequences resynthesised using synthetic oligonucleotides. This is important as it also allows the human-derived gene sequence to be codon-optimised for *E. coli* in order to improve protein expression. Dominant VH and/or VL sequences can be spliced together using a spacer (linker) sequence. This gene cassette, termed a scFv, can be resynthesised to include terminal *Nde*I and *Not*I restriction sites for cloning into expression vector pET29b (Novagen). ScFv DNA can be cut with *Nde*I (cuts in VH) or *Not*I (cuts in VL) using the following reaction: 40 µl DNA (4 µg), 10 µl of Restriction enzyme buffer D (Promega), 47 µl water and 2 µl NdeI and NotI enzyme (10 u µl⁻¹; Promega) with digestion for 4 h at 37 °C. DNA can be then fractionated on 0.7% agarose TAE gels and the digested DNA excised from the gel and purified from the agarose slice using the Geneclean II kit (Bio 101) exactly according to the manufacturers instruction. pET29b vector DNA can be cut with *Nde*I and *Not*I as described above. 1 µg vector can be mixed with 1 µg restricted VL DNA resuspended in 8.5 µl water and ligated by addition of 1 µl 10 x ligation buffer (Boehringer Mannheim) and 0.5 µl DNA ligase (3 u µl⁻¹; Boehringer Mannheim), followed by ligation overnight at 14 °C.

The entire ligation can be transformed into *E. coli* TG2 cells (Stratagene, UK) by electroporation. Transformants can be plated out onto agar plates containing Tetracycline (25 µg ml⁻¹ - selective for recA ::Tn10 on the chromosome of TG2) and Kanamycin (50 µg ml⁻¹ - selective for pET29). Recombinant plasmid DNA prepared from individual clones can be checked for scFv sequence by digestion with *Nde*I and *Not*I as described above.

Recombinant scFvs can be over-expressed in *E. coli* strain JM109(DE3) and purified thus enabling biochemical and biological characterisation. Recombinants can be spread on LB plates supplemented with 50 µg ml⁻¹ kanamycin and a single colony can be used to inoculate a 10 microlitre starter culture of LB broth with 50 µg ml⁻¹ kanamycin. For this, a 1 L expression culture can be prepared in the presence of kanamycin for 4-5 h at 37 °C with shaking at 300 rpm. At OD₆₀₀ =1, induction can be performed using IPTG and the cells can be grown with vigorous aeration for a further 3-5 h. Cells can then be harvested by centrifugation (4000 x g, 15 minutes, 4 °C). The cell pellet can be resuspended in 10 ml fresh (4 °C) lysis buffer (below) and stored at -20 °C overnight prior to cell breakage using the 25 ml X-Press system (AB Biox)

### LYSIS BUFFER

50 mM Tris HCl pH 8.0
1 mM EDTA
100 mM KCl
0.1 mM AEBSF (amino ethyl benzene sulphonic acid)

The lysate can be centrifuged in an Oakridge tube (24 300 x g, 4 °C, 30 minutes). The pellet can be resuspended in 20 ml of ice cold lysis buffer on ice using a Silverson lab blender. The lysate can be split into 8 x Oakridge tubes and each aliquot can be diluted to 30 ml (total 120 ml) in ice-cold Lysis Buffer. Inclusion bodies can be pelleted (24 300 x g, 4 °C, 30 minutes) and the supernatant discarded. Each of the 8 pellets can be resuspended in 30 ml of lysis buffer, and the inclusion bodies can be harvested by centrifugation again (24 300 x g, 4 °C, 30 minutes). This wash/centrifugation step can be performed five times in total to clean the inclusion body fraction. Each pellet can be resuspended in 15 ml of ice-cold water and the inclusion bodies can be stored at -20 °C.

For refolding, 200 ml solution of 2% (w/v) N-lauryl sarcosine (NLS) in 50 mM Tris HCl pH 9.0 can be prepared as follows. 4 g NLS can be solubilised in 100 ml water with stirring. 10 ml of 1M Tris pH 9.0 stock solution can be added and made up to 195 ml with water. 5 ml of inclusion body slurry can be added and stirred vigorously for 30 minutes at room temperature.

CuCl₂ can be added to a concentration of 100 µM. This serves as a catalyst for oxidation. The refolding reaction can be transferred to 4 °C and stirred vigorously for 2 days to promote aeration. The refolding reaction can be vacuum filtered through a 0.44 µM vacucup bottle top filter unit (90 mm diameter, Gellman Sciences) and the filtrate transferred to a Pellicon Labscale TFF system fitted with PLGC10 membrane unit (Millipore). The reaction can be concentrated to 25 ml using tangential flow, discarding the permeate (the scFv is localised to the retentate). The solution can be diafiltered against 40 x turn-over volumes (1 L) of 10 mM ammonium acetate (AAT) pH 9.0. Finally, the volume of the antibody can be diluted to 50 ml using 10 mM AAT pH 9.0. The buffer exchanged antibody can be stored for 2 hours at 4 °C. The typical protein content was 1 - 2 mg ml⁻¹ with a yield of up to 50 mg per litre.

For scFv purification a 10 ml bed volume of Ni NTA superflow agarose Qiagen can be prepared according to the manufacturers instructions using a 10 ml glass column (Sigma) without flow adaptors. The column can be equilibrated with 50 ml Buffer B (6M urea, 0.1 M NaH₂PO₄,10 mM Tris HCl pH 8.0). Refolded scFv (as described above)can be diluted 1/5 in Buffer B and applied directly to the column at ml min⁻¹. 50 ml buffer B can be applied to the column (flow rate 5 ml min⁻¹) followed by 50 ml Buffer C (same composition as buffer B but pH 6.3). The purified scFv can be eluted from the column by slowly applying Buffer B supplemented with 250 mM high grade imidazole until the A₂₈₀ < 0.05.

### Results

The variable heavy chain (VH) and variable light chains of the antibody genes from peripheral blood lymphocytes (PBLs) isolated from *C. difficile* infected patients have been TA cloned and sequenced. In each case the third complementarity determining region (i.e. CDR-H3 or CDR-L3) was identified. This region is both the most variable region of the VH chain and the area identified as being most important in determining antigen binding, for this reason this area is used as the signature for each VH chain.

### Clinical Information

Patient D01 - Variable heavy chains (VH's) and variable light chains (VL's) were sequenced from PBLs taken ten days after the onset of CDAD. Lab results confirmed *C. difficile* toxin was present.

Patient D02 - VH's and VL's were sequenced from PBLs taken ten days after the onset of CDAD. Lab results confirmed *C. difficile* toxin was present. In the seven subsequent weeks after sample was taken, the patient did not develop any further *C. difficile* infections, suggesting that the antibodies produced in response to the infection were protective.

Patient D03 - VH's and VL's were sequenced from PBLs taken ten days after the onset of CDAD. Lab results confirmed *C. difficile* toxin was present. In the three subsequent weeks after sample was taken, the patient did not develop any further *C. difficile* infections, suggesting that the antibodies produced in response to the infection were protective.

### Sequencing results

Patient D01 showed a high degree of focusing with both VH's and VL's. Three different CDR3 sequences make-up over 80 % of the sequenced VH's. VH's with the CDR3 sequence of SEQ ID NO: 27 account for 57.8 % of sequenced VH's from this patient. VH's with the CDR3 sequence of SEQ ID NO: 28 account for 13.5 % of all VH's, and VH's the CDR3 sequence of SEQ ID NO: 29 account for 10 % of all sequenced VH's in this patient. Three different CDR3 sequences also make up the majority of sequenced VL's, these are the CDR3 sequences of SEQ ID NOs: 32, 34, and 33 (occurring at frequencies of 32.7 %, 15.3%, and 6.6%, respectively).

Similarity searches were performed using these common VH and/or VL sequences against other VH and/or VL sequences contained within a database (FABTEC database). which contains VH and/or VL regions of PBLs isolated from patients with methicillin-resistant *Staphylococcus aureus* (MRSA), *Candida albicans, Pseudomonas aeruginosa* (the causative agent of cystic fibrosis), *Streptococcus oralis* and vancomycin-resistant Enterococci (VRE) infections and to identify antibody sequences which are associated with resistance to the infections and which can therefore be used to effect therapy against those infections. These sequences have formed the basis of WO 01/76627. For all of these, the isolated VH and/or VL sequences demonstrate skewing of the antibody repertoire over the course of infection, thus revealing the identity of matured, immunity-conferring VH and/or VL sequences. This has typically been done using 5,000 to 15,000 sequences for each VH and/or VL. VH and/or VL repertoires from healthy individuals can also be used for comparison purposes.

Using the BLOSUM50 matrix it was possible to pick out groups of antibodies with CDR3's that had greater than 70% similarity. The CDR3 sequences of SEQ ID NOs: 27 and 28 (Table 2 and 3, respectively), showed sequence similarity of greater than 70% only with other VH's from *C. difficile* infected patients VH's sequenced from individuals with other infections (*C. albicans,* MRSA and VRE) showed no similar CDR3 sequences.

In the following Tables, libraries starting D are derived from *C. difficile* infected patients, libraries starting M are derived from MRSA infected patients, libraries starting V are derived from VRE infected patients; and libraries starting C are derived from *C. albicans* infected patients.

**Table 2**

| CDR3 SEQ ID NO: | Similarity | Library | No. of clones |
|---|---|---|---|
| 27 | 100.0 | D01 | 184 |
| 27 | 100.0 | D03 | 5 |
| 35 | 100.0 | D01 | 1 |
| 36 | 100.0 | D01 | 3 |
| 37 | 100.0 | D01 | 1 |
| 37 | 100.0 | D03 | 1 |
| 38 | 93.75 | D01 | 1 |

**Table 3**

| CDR3 SEQ ID NO: | Similarity | Library | No. of clones |
|---|---|---|---|
| 28 | 100.0 | D01 | 43 |
| 28 | 100.0 | D03 | 1 |
| 39 | 100.0 | D01 | 1 |
| 40 | 88.88 | D01 | 1 |

VH's with the CDR3 sequence of SEQ ID NO: 29 showed similarity to VH's from other *C*. *difficile* infected patients and MRSA infected patients (Table 4).

**Table 4**

| CDR3 SEQ ID NO: | Similarity | Library | No. of clones |
|---|---|---|---|
| 41 | 100.0 | D01 | 1 |
| 29 | 100.0 | D01 | 32 |
| 29 | 100.0 | M01 | 1 |
| 29 | 100.0 | M02 | 5 |
| 29 | 100.0 | M03 | 2 |
| 29 | 100.0 | M04 | 8 |
| 42 | 100.0 | M03 | 1 |
| 43 | 100.0 | D01 | 1 |
| 44 | 93.75 | D01 | 1 |
| 45 | 93.75 | D01 | 1 |
| 46 | 75.0 | M04 | 1 |
| 47 | 70.58 | M05 | 1 |

The CDR3 sequences from the VL's of patient D01 have also had a similarity search performed on them. VL's with the CDR3 sequences of SEQ ID NOs: 32 and 33 (Tables 5 and 6, respectively) show a high degree of homology with CDR3 sequences from *C. difficile* infected patients but no homology to CDR3 sequences derived from patients with different clinical infections. VL's with the CDR3 sequence of SEQ ID NO: 34 showed similarity to VL's isolated from *C. difficile,* MRSA and VRE infected patients (Table 7).

**Table 5**

| CDR3 SEQ ID NO: | Similarity | Library | No. of clones |
|---|---|---|---|
| 32 | 100.0 | D51 | 64 |
| 48 | 90.9 | D52 | 2 |
| 49 | 90.9 | D52 | 1 |
| 50 | 90.9 | D52 | 1 |
| 51 | 90.0 | D51 | 1 |
| 52 | 90.0 | D52 | 1 |
| 53 | 81.8 | D52 | 1 |
| 54 | 81.8 | D52 | 1 |
| 55 | 81.8 | D52 | 2 |
| 56 | 81.8 | D52 | 1 |
| 57 | 80.0 | D52 | 1 |
| 58 | 72.7 | D52 | 1 |

**Table 6**

| CDR3 SEQ ID NO: | Similarity | Library | No. of clones |
|---|---|---|---|
| 33 | 100.0 | D51 | 13 |
| 59 | 90.9 | D52 | 1 |
| 60 | 83.3 | D52 | 1 |
| 61 | 81.8 | D52 | 4 |
| 62 | 81.8 | D52 | 1 |
| 63 | 81.8 | D52 | 1 |
| 64 | 81.8 | D52 | 1 |
| 65 | 81.8 | D51 | 1 |
| 66 | 81.8 | D52 | 1 |
| 67 | 81.8 | D52 | 1 |
| 68 | 72.7 | D52 | 1 |
| 69 | 72.7 | D52 | 1 |

**Table 7**

| CDR3 SEQ ID NO: | Similarity | Library | No. of clones |
|---|---|---|---|
| 34 | 100.0 | D51 | 30 |
| 34 | 100.0 | M56 | 2 |
| 34 | 100.0 | V54 | 2 |
| 70 | 88.9 | D51 | 1 |
| 71 | 88.9 | D51 | 1 |
| 72 | 88.9 | V54 | 2 |
| 73 | 77.9 | M55 | 1 |
| 74 | 77.9 | D51 | 1 |
| 75 | 77.9 | M55 | 1 |
| 76 | 77.9 | M55 | 1 |

Patient D02 also showed a high degree of focusing of the VH repertoire, although focusing is less obvious in the VL repertoire. Two types predominate in the VH library of this patient. VH's with the CDR3 sequence of SEQ ID NO: 30 accounted for 45.6% of sequenced clones and those with the CDR3 sequence of SEQ ID NO: 31 accounted for 8.8% of the library. In the VL library the three most frequently isolated clones with the CDR3 sequences of SEQ ID NOs: 61, 96, and 97 were found at 4%, 4% and 3%, respectively.

Similarity searches in the FABTEC database showed the two common VH CDR3 sequences to be specific to *C. difficile* patients (Tables 8 and 9). VH's with the CDR3 sequence of SEQ ID NO: 30 match with other antibodies in that library and antibodies from library D03 (Table 8). VH's with the CDR3 sequence of SEQ ID NO: 31 match with antibodies from library D03 (Table 9).

**Table 8**

| CDR3 SEQ ID NO: | Similarity | Library | No. of clones |
|---|---|---|---|
| 77 | 100.0 | D02 | 1 |
| 78 | 100.0 | D02 | 1 |
| 30 | 100.0 | D02 | 52 |
| 30 | 100.0 | D03 | 1 |
| 79 | 100.0 | D02 | 1 |
| 79 | 100.0 | D03 | 1 |
| 80 | 94.4 | D02 | 1 |
| 81 | 94.4 | D02 | 1 |
| 82 | 94.4 | D02 | 1 |
| 83 | 88.8 | D02 | 1 |
| 84 | 77.7 | D03 | 1 |

**Table 9**

| CDR3 SEQ ID NO: | Similarity | Library | No. of clones |
|---|---|---|---|
| 31 | 100.0 | D02 | 10 |
| 31 | 100.0 | D03 | 4 |
| 85 | 90.0 | D03 | 1 |
| 86 | 90.0 | D03 | 1 |

Similarity searches using some of the VL's showed a clustering of sequences. 15% of VL's from patient D02 (library D52) showed high degree of similarity to the CDR3 sequence of SEQ ID NO: 48, VL's from patient D01 (library D51) also showed a high degree of homology to this sequence (Table 10). In a similar way a cluster around the sequence of SEQ ID NO: 61 accounts for 13% of patient D02 sequenced VL's, again clones similar to this one are also found in patient D01 (Table 11). Neither of these clones show any significant similarity to VL CDR3 sequences isolated from patients infected with other pathogens. It should also be note that using the BLOSUM50 matrix these two clones show greater than 60% similarity to each other.

**Table 10**

| CDR3 SEQ ID NO: | Similarity | Library | No. of clones |
|---|---|---|---|
| 48 | 100.0 | D52 | 2 |
| 49 | 100.0 | D52 | 1 |
| 53 | 100.0 | D52 | 1 |
| 32 | 90.9 | D51 | 64 |
| 87 | 81.8 | D52 | 2 |
| 51 | 81.8 | D51 | 1 |
| 88 | 81.8 | D52 | 1 |
| 57 | 72.7 | D52 | 1 |
| 89 | 72.7 | D52 | 1 |
| 50 | 72.7 | D52 | 1 |
| 54 | 72.7 | D52 | 1 |
| 55 | 72.7 | D52 | 2 |

**Table 11**

| CDR3 SEQ ID NO: | Similarity | Library | No. of clones |
|---|---|---|---|
| 61 | 100.0 | D52 | 4 |
| 65 | 100.0 | D51 | 1 |
| 59 | 81.8 | D52 | 1 |
| 33 | 81.8 | D51 | 13 |
| 62 | 81.8 | D52 | 1 |
| 63 | 81.8 | D52 | 1 |
| 60 | 75.0 | D52 | 1 |
| 64 | 72.7 | D52 | 1 |
| 66 | 72.7 | D52 | 1 |
| 68 | 72.7 | D52 | 1 |
| 67 | 72.7 | D52 | 1 |

Patient D03 has only preliminary results for VH's but shows many similar sequences to both D01 and D02 so are worth reporting. Only data from 49 VH sequences have been produced. 10% of the clones have the CDR3 sequence of SEQ ID NO: 27, this is the sequence of the most common VH in patient D01. 8% of VH's have the CDR3 sequence of SEQ ID NO: 31 - this is the second most common VH CDR3 sequence in patient D02. Cross-Library comparison (Table 12) of the CDR3 sequences of VH's has shown 8 CDR3 sequences which appear in more than one of the *C. difficile* infected patients. The CDR3 sequences of SEQ ID NOs: 27, 28, and 37 appear in patients D01 and D03 and the sequences of SEQ ID NOs: 31, 90, 91, 79, and 30 all appear in patients D02 and D03. So far patients D01 and D02 show no common CDR3 sequences. VL's have only been sequenced from patients D01 and D02, these patients share no identical CDR3 sequences.

**Table 12**

| VH CDR3 SEQ ID NO: | Patient |
|---|---|
| 27 | D01 and D03 |
| 28 | D01 and D03 |
| 37 | D01 and D03 |
| 31 | D02 and D03 |
| 90 | D02 and D03 |
| 91 | D02 and D03 |
| 79 | D02 and D03 |
| 30 | D02 and D03 |

### Summary

The presence of a large number of a particular VH CDR3 sequence in a patient with a *C. difficile* infection indicates that VH as part of an scFv may be protective against the organism. CDR3 sequences that are shared by more than one patient indicate specific *C. difficile* sequences. It should also be noted that sequences with a high degree of homology often due to somatic mutation within the patient may also represent important sequences with similar but subtly different properties. Analysis of the CDR3 sequences of the VH from our individuals has lead to the identification of 17 potentially protective VH's. These VH CDR3 sequences correspond to SEQ ID NOs: 30, 79, 28, 91, 41, 39, 38, 27, 36, 36, 40, 37, 90, 29, 44, 45, and 43.

A similar analysis of the VL has identified 44 different VL CDR3 sequences. The larger number is due to the increased rate of somatic mutation in antibody light chains leading to VL's with slightly different sequences. These VL CDR3 sequences correspond to SEQ ID NOs: 59, 60, 61, 33, 62, 63, 64, 65, 66, 77, 84, 78, 30, 79, 80, 81, 82, 87, 57, 74, 85, 31, 86, 82, 51, 48, 88, 49, 53, 32, 52, 70, 71, 34, 89, 68, 67, 50, 54, 55, 56, 59, 58, 96, and 97.

## Claims

1. A method for identifying candidate sequences of at least the CDR3 region of antibodies specific against at least one antigen produced by *Clostridium difficile* during an infection or against a vaccine, comprising the steps of:
(i) with B cells isolated from at least one patient who has been infected by *Clostridium difficile* or administered said vaccine, sequencing at least the CDR3 region of the VH and/or VL coding regions of said B cells; and
(ii) correlating said sequences of at least the CDR3 regions of the VH and/or VL coding regions of said B cells from said at least one patient to identify a set of candidate sequences for at least a CDR3 region of antibodies specific against said at least one antigen produced by *Clostridium difficile* or against said vaccine, each of said set of candidate CDR3 sequences or a sequence having at least 80% homology therewith occurring in total at a frequency of at least 1 percent in the set of sequences determined at step (i).

2. A method according to claim 1, said B cells being selected from the group consisting of peripheral B-cell lymphocytes and B cells from the spleen.

3. A method according to claim 2, said peripheral B-cell lymphocytes being isolated from blood from said at least one patient.

4. A method according to any of claims 1-3, said at least one antigen being an immunogen.

5. A method according to any of claims 1-4, said at least one patient displaying a pronounced antibody response in response to infection by *Clostridium difficile*.

6. A method according to any of claims 1-5, said at least one patient having recovered from infection by *Clostridium difficile*.

7. A method according to any of claims 1-6, said correlation step (ii) comprising determining putative amino acid sequences from said sequences of at least the VH and/or VL CDR3 coding regions, and correlating said putative amino acid sequences.

8. A method according to claim 6, said correlation step (ii) comprising identifying the Complementarity Determining Regions comprised in said at least the VH and/or VL regions and correlating said Complementarity Determining Regions.

9. A method according to claim 8, said Complementarity Determining Regions being selected from the group consisting of CDR1, CDR2 and CDR3.

10. A method according to any of claims 1-9, said correlation step (ii) additionally correlating at least one of the group consisting of: the strain of *Clostridium difficile* infecting said at least one patient, the time point at which said B cells are isolated during infection of said at least one patient by *Clostridium difficile*, the age of said at least one patient, the sex of said at least one patient, and the race of said at least one patient.

11. A method according to any of claims 1-10, said B cells having been isolated from said at least one patient at a plurality of time points during infection of said at least one patient by *Clostridium difficile*, said correlation step (ii) correlating the time point during infection of said at least one patient by *Clostridium difficile* at which said B cells are isolated.

12. A method according to any of claims 1-10, said B cells having been isolated from at least two patients, at least one of whom has recovered from infection by *Clostridium difficile*, and at least one of whom has not recovered from infection by *Clostridium difficile*, said correlation step (ii) correlating the recovery of said at least two patients from infection by *Clostridium difficile*.

13. A method according to any of claims 1-10, said B cells having been isolated from at least two patients, said patients being infected by different strains of *Clostridium difficile* producing said at least one antigen, said correlation step (ii) correlating said sequences of at least the VH and/or VL coding regions of said B cells to identify a set of candidate sequences for antibodies, each of which is specific against at least one shared antigen produced by said different strains of *Clostridium difficile* or is specific against different antigens produced by said different strains of *Clostridium difficile*.

14. A method of producing a database which identifies candidate sequences for antibodies specific against at least one antigen produced by *Clostridium difficile*, comprising the steps of:
(i) performing a method according to any of claims 1-13; and
(ii) storing the data produced by said method in said database.

15. A method according to claim 14 further comprising the step of:
(iii) producing a report comprising the data produced by said method.

## Patentansprüche

1. Methode zum Identifizieren von Kandidatensequenzen von zumindest der CDR3-Region von Antikörpern, die gegen mindestens ein Antigen, das durch *Clostridium difficile* während einer Infektion erzeugt wird, oder gegen einen Impfstoff spezifisch sind, umfassend die Schritte des:
(i) Sequenzierens bei B-Zellen, die von mindestens einem Patienten isoliert worden sind, der durch *Clostridium difficile* infiziert worden ist oder dem der Impfstoff verabreicht worden ist, zumindest der CDR3-Region der VH und/oder VL kodierenden Regionen der B-Zellen; und
(ii) Korrelierens der Sequenzen zumindest der CDR3-Regionen der VH und/oder VL kodierenden Regionen der B-Zellen von mindestens einem Patienten, um einen Satz von Kandidatensequenzen für mindestens eine CDR3-Region von Antikörpern zu identifizieren, die gegen das mindestens eine Antigen, das durch *Clostridium difficile* erzeugt wird, oder gegen den Impfstoff spezifisch sind, wobei jeder Satz Kandidaten-CDR3-Sequenzen oder Sequenzen, die mindestens eine 80 %ige Homologie damit aufweisen, insgesamt mit einer Häufigkeit von mindestens 1 Prozent in dem in Schritt (i) bestimmten Satz von Sequenzen auftritt.

2. Methode nach Anspruch 1, wobei die B-Zellen aus der Gruppe ausgewählt werden bestehend aus peripheren B-Zellen-Lymphozyten und B-Zellen aus der Milz.

3. Methode nach Anspruch 2, wobei die peripheren B-Zellen-Lymphozyten aus dem Blut des mindestens einen Patienten isoliert werden.

4. Methode nach einem der Ansprüche 1-3, wobei mindestens ein Antigen ein Immunogen ist.

5. Methode nach einem der Ansprüche 1-4, wobei der mindestens eine Patient als Reaktion auf die Infektion durch *Clostridium difficile* hin eine ausgeprägte Antikörper-Reaktion aufweist.

6. Methode nach einem der Ansprüche 1-5, wobei der mindestens eine Patient von der Infektion durch *Clostridium difficile* rekonvalesziert ist.

7. Methode nach einem der Ansprüche 1-6, wobei der Korrelationsschritt (ii) das Bestimmen mutmaßlicher Aminosäuresequenzen aus den Sequenzen zumindest der VH- und/oder VL-CDR3 kodierenden Regionen und das Korrelieren der mutmaßlichen Aminosäuresequenzen umfasst.

8. Methode nach Anspruch 6, wobei der Korrelationsschritt (ii) das Identifizieren der komplementären Bestimmungsregionen, die zumindest in den VH- und/oder VL-Regionen umfasst sind, und das Korrelieren der komplementären Bestimmungsregionen umfasst.

9. Methode nach Anspruch 8, wobei die komplementären Bestimmungsregionen aus der Gruppe ausgewählt werden bestehend aus CDR1, CDR2 und CDR3.

10. Methode nach einem der Ansprüche 1-9, wobei der Korrelationsschritt (ii) zusätzlich mindestens eine der Gruppen korreliert bestehend aus: dem Stamm *Clostridium difficile*, der mindesten einen Patienten infiziert, dem Zeitpunkt, an dem die B-Zellen während der Infektion des mindestens einen Patienten durch *Clostridium difficile* isoliert werden, dem Alter des mindestens einen Patienten, dem Geschlecht des mindestens einen Patienten und der Rasse des mindestens einen Patienten.

11. Methode nach einem der Ansprüche 1-10, wobei die B-Zellen aus dem mindestens einen Patienten zu mehreren Zeitpunkten während der Infektion des mindestens einen Patienten durch *Clostridium difficile* isoliert worden sind, wobei der Korrelationsschritt (ii) den Zeitpunkt während der Infektion des mindesten einen Patienten durch *Clostridium difficile* korreliert, an dem die B-Zellen isoliert werden.

12. Methode nach einem der Ansprüche 1-10, wobei die B-Zellen aus mindestens zwei Patienten isoliert worden sind, von denen mindestens einer von der Infektion durch *Clostridium difficile* rekonvalesziert ist, und mindestens einer von ihnen nicht von der Infektion durch *Clostridium difficile* rekonvalesziert ist, wobei der Korrelationsschritt (ii) die Rekonvaleszierung der mindestens zwei Patienten von der Infektion durch *Clostridium difficile* korreliert,

13. Methode nach einem der Ansprüche 1-10, wobei die B-Zellen aus mindestens zwei Patienten isoliert worden sind, wobei die Patienten durch verschiedene Stämme von *Clostridium difficile,* die das mindestens eine Antigen erzeugen, infiziert sind, wobei der Korrelationsschritt (ii) die Sequenzen von mindestens den VH und/oder VL kodierenden Regionen der B-Zellen korrelieren, um einen Satz von Kandidatensequenzen für Antikörper zu identifizieren, von denen jeder gegen mindestens ein gemeinsames Antigen, das durch die verschiedenen Stämme von *Clostridium difficile* erzeugt wird, oder gegen verschiedene Antigene spezifisch ist, die durch die verschiedenen Stämme von *Clostridium difficile* erzeugt werden.

14. Methode zum Herstellen einer Datenbase, die Kandidatensequenzen für Antikörper identifiziert, die gegen mindestens ein Antigen spezifisch sind, das durch *Clostridium difficile* erzeugt wird, umfassend die Schritte des:
(i) Durchführens einer Methode nach einem der Ansprüche 1-13; und
(ii) Speichern der durch die Methode erzeugten Daten in der Datenbase.

15. Methode nach Anspruch 14, des Weiteren den Schritt umfassend des:
(iii) Erstellens eines Berichts umfassend die durch die Methode erzeugten Daten.

## Revendications

1. Méthode d'identification de séquences candidates d'au moins la région CDR3 d'anticorps spécifiques contre au moins un antigène produit par *Clostridium difficile* pendant une infection ou contre un vaccin, comprenant les étapes consistant à:
(i) avec des cellules B isolées d'au moins un patient qui a été infecté par *Clostridium difficile* ou auquel ledit vaccin a été administré, séquencer au moins la région CDR3 des régions codantes VH et/ou VL desdites cellules B; et
(ii) mettre en corrélation lesdites séquences d'au moins les régions CDR3 des régions codantes VH et/ou VL desdites cellules B dudit au moins un patient afm d'identifier une série de séquences candidates pour au moins une région CDR3 d'anticorps spécifiques contre ledit au moins un antigène produit par *Clostridium difficile* ou contre ledit vaccin, chacune de ladite série de séquences candidates de CDR3 ou une séquence ayant au moins 80% d'homologie avec celle-ci, survenant au total à une fréquence d'au moins 1 pour cent dans la série de séquences déterminée à l'étape (i).

2. Méthode selon la revendication 1, lesdites cellules B étant sélectionnées parmi le groupe consistant en cellules lymphocytaires B périphériques et en cellules B de la rate.

3. Méthode selon la revendication 2, lesdites cellules lymphocytaires B périphériques étant isolées du sang dudit au moins un patient.

4. Méthode selon l'une quelconque des revendications 1-3, ledit au moins un antigène étant un immunogène.

5. Méthode selon l'une quelconque des revendications 1-4, ledit au moins un patient présentant une réponse prononcée d'anticorps en réponse à une infection à *Clostridium difficile*.

6. Méthode selon l'une quelconque des revendications 1-5, ledit au moins un patient s'étant rétabli d'une infection à *Clostridium difficile*.

7. Méthode selon l'une quelconque des revendications 1-6, ladite étape de corrélation (ii) comprenant déterminer des séquences d'acides aminés potentielles desdites séquences d'au moins les régions codantes CDR3 des VH et/ou VL, et mettre les séquences d'acides aminés potentielles en corrélation.

8. Méthode selon la revendication 6, ladite étape de corrélation (ii) comprenant identifier les régions déterminantes complémentaires comprises dans au moins lesdites régions codantes VH et/ou VL et mettre lesdites régions déterminantes complémentaires en corrélation.

9. Méthode selon la revendication 8, lesdites régions déterminantes complémentaires étant sélectionnées parmi le groupe consistant en CDR1, CDR2 et CDR3.

10. Méthode selon l'une quelconque des revendications 1-9, ladite étape de corrélation (ii) mettant en plus en corrélation au moins un élément du groupe consistant en: la souche de *Clostridium difficile* infectant ledit au moins un patient, le point temporel auquel lesdites cellules B sont isolées durant l'infection par *Clostridium difficile* dudit au moins un patient, l'âge dudit au moins un patient, le sexe dudit au moins un patient et la race dudit au moins un patient.

11. Méthode selon l'une quelconque des revendications 1-10, lesdites cellules B ayant été isolées dudit au moins un patient à une pluralité de points temporels durant l'infection par *Clostridium difficile* dudit au moins un patient, ladite étape de corrélation (ii) mettant en corrélation le point temporel durant l'infection par *Clostridium difficile* dudit au moins un patient, auquel lesdites cellules B sont isolées.

12. Méthode selon l'une quelconque des revendications 1-10, lesdites cellules B ayant été isolées d'au moins deux patients, l'un au moins desquels s'est rétabli d'une infection à *Clostridium difficile* et l'un au moins desquels ne s'est pas rétabli d'une infection à *Clostridium difficile*, ladite étape de corrélation (ii) mettant en corrélation le rétablissement desdits au moins deux patients d'une infection à *Clostridium difficile*.

13. Méthode selon l'une quelconque des revendications 1-10, lesdites cellules B ayant été isolées d'au moins deux patients, lesdits patients étant infectés par des souches différentes de *Clostridium difficile* produisant ledit au moins un antigène, ladite étape de corrélation (ii) mettant en corrélation lesdites séquences d'au moins les régions codantes VH et/ou VL desdites cellules B, afin d'identifier une série de séquences candidates pour des anticorps, dont chacun desquels est spécifique contre au moins un antigène partagé produit par lesdites différentes souches de *Clostridium difficile,* ou est spécifique contre des antigènes différents produits par lesdites différentes souches de *Clostridium difficile*.

14. Méthode de production d'une base de données qui identifie des séquences candidates pour des anticorps spécifiques contre au moins un antigène produit par *Clostridium difficile*, comprenant les étapes consistant à:
(i) exécuter la méthode selon l'une quelconque des revendications 1-13; et
(ii) stocker les données produites par ladite méthode dans ladite base de données.

15. Méthode selon la revendication 14, comprenant en outre l'étape consistant à:
(iii) produire un rapport comprenant les données produites par ladite méthode.


### SEQUENCE LISTING

<110> NeuTec Pharma PLC
<120> Clostridium difficile Focussed Antibodies
<130> GDM/JR-MP100462-WO
<150> GB0309126.1
   <151> 2003-04-17
<160> 97
<170> PatentIn version 3.2
<210> 1
   <211> 24
   <212> DNA
   <213> Artificial Sequence
<220>
   <223> PCR Primer
<400> 1
   gtccaccttg gtgttgctgg gctt 24
<210> 2
   <211> 24
   <212> DNA
   <213> Artificial Sequence
<220>
   <223> PCR Primer
<400> 2
   agactctccc ctgttgaagc tctt 24
<210> 3
   <211> 24
   <212> DNA
   <213> Artificial Sequence
<220>
   <223> PCR Primer
<400> 3
   tgaggagacg gtgaccaggg tgcc 24
<210> 4
   <211> 24
   <212> DNA
   <213> Artificial Sequence
<220>
   <223> PCR Primer
<400> 4
   tgaagagacg gtgaccattg tccc 24
<210> 5
   <211> 24
   <212> DNA
   <213> Artificial Sequence
<220>
   <223> PCR Primer
<400> 5
   tgaggagacg gtgaccaggg ttcc 24
<210> 6
   <211> 24
   <212> DNA
   <213> Artificial Sequence
<220>
   <223> PCR Primer
<400> 6
   tgaggagacg gtgaccgtgg tccc 24
<210> 7
   <211> 23
   <212> DNA
   <213> Artificial Sequence
<220>
   <223> PCR Primer
<400> 7
   caggtgcagc tggtgcagtc tgg 23
<210> 8
   <211> 23
   <212> DNA
   <213> Artificial Sequence
<220>
   <223> PCR Primer
<400> 8
   caggtgaact taagggagtc tgg 23
<210> 9 <211> 23
   <212> DNA
   <213> Artificial Sequence
<220>
   <223> PCR Primer
<400> 9
   caggtgcagc tggtggagtc tgg 23
<210> 10
   <211> 23
   <212> DNA
   <213> Artificial Sequence
<220>
   <223> PCR Primer
<400> 10
   caggtgcagc tgcaggagtc ggg 23
<210> 11
   <211> 23
   <212> DNA
   <213> Artificial Sequence
<220>
   <223> PCR Primer
<400> 11
   caggtgcagc tacagcagtg ggg 23
<210> 12
   <211> 23 <212> DNA
   <213> Artificial Sequence
<220>
   <223> PCR Primer
<400> 12
   gaggtgcagc tgttgcagtc tgc 23
<210> 13
   <211> 23
   <212> DNA
   <213> Artificial Sequence
<220>
   <223> PCR Primer
<400> 13
   caggtacagc tgcagcagtc agg 23
<210> 14
   <211> 24
   <212> DNA
   <213> Artificial Sequence
<220>
   <223> PCR Primer
<400> 14
   acgtttgatt tccaccttgg tccc 24
<210> 15
   <211> 24
   <212> DNA <213> Artificial Sequence
<220>
   <223> PCR Primer
<400> 15
   acgtttgatc tccagcttgg tccc 24
<210> 16
   <211> 24
   <212> DNA
   <213> Artificial Sequence
<220>
   <223> PCR Primer
<400> 16
   acgtttgata tccactttgg tccc 24
<210> 17
   <211> 24
   <212> DNA
   <213> Artificial Sequence
<220>
   <223> PCR Primer
<400> 17
   acgtttgatc tccaccttgg tccc 24
<210> 18
   <211> 24
   <212> DNA
   <213> Artificial Sequence
<220>
   <223> PCR Primer
<400> 18
   acgtttattc tccagtcgtg tccc 24
<210> 19
   <211> 23
   <212> DNA
   <213> Artificial Sequence
<220>
   <223> PCR Primer
<400> 19
   gacatccaga tgacccagtc tcc 23
<210> 20
   <211> 23
   <212> DNA
   <213> Artificial Sequence
<220>
   <223> PCR Primer
<400> 20
   gatgttgtga tgactcagtc tcc 23
<210> 21
   <211> 23
   <212> DNA
   <213> Artificial Sequence
<220>
   <223> PCR Primer
<400> 21
   gaaattgtgt tgacgcagtc tcc 23
<210> 22
   <211> 23
   <212> DNA
   <213> Artificial Sequence
<220>
   <223> PCR Primer
<400> 22
   gacatcgtga tgacccagtc tcc 23
<210> 23
   <211> 23
   <212> DNA
   <213> Artificial Sequence
<220>
   <223> PCR Primer
<400> 23
   gaaactacac tcacgcagtc tcc 23
<210> 24
   <211> 23
   <212> DNA
   <213> Artificial Sequence
<220>
   <223> PCR primer
<400> 24
   gaaattgtgc tgactcagtc tcc 23
<210> 25
   <211> 17
   <212> DNA
   <213> Artificial Sequence
<220>
   <223> PCR Primer
<400> 25
   gtaaaacgac ggccagt 17
<210> 26
   <211> 16
   <212> DNA
   <213> Artificial Sequence
<220>
   <223> PCR Primer
<400> 26
   aacagctatg accatg 16
<210> 27
   <211> 16
   <212> PRT
   <213> Homo sapiens
<400> 27
   Glu Ile Arg Ala Pro Asp His His Asp Phe Ser Gly Tyr Leu Gly Arg
   1               5                   10                  15
<210> 28
   <211> 9
   <212> PRT
   <213> Homo sapiens
<400> 28
   Asp Ile Ser Ala Gly Gly Leu Asp Val
   1               5
<210> 29
   <211> 16
   <212> PRT
   <213> Homo sapiens
<400> 29
   Asn Val Gly Ser Gly Ser Tyr Tyr Thr Gly Gly His Trp Phe Asp Pro
   1               5                   10                  15
<210> 30
   <211> 18
   <212> PRT
   <213> Homo sapiens
<400> 30
   Asp Gly Val Arg Gln Tyr Ser Gly Gly Arg Tyr Ser Asn His Gly Met
   1 5 10 15
   Asp Val
<210> 31
   <211> 10
   <212> PRT
   <213> Homo sapiens
<400> 31
   Leu Thr Ala Ala Gly Gly His Phe Asp Pro
   1 5 10
<210> 32
   <211> 10
   <212> PRT
   <213> Homo sapiens
<400> 32
   Asn Ser Arg Asp Ser Thr Gly Asn Gln Leu
   1 5 10
<210> 33
   <211> 11
   <212> PRT
   <213> Homo sapiens
<400> 33
   Ala Ala Trp Asp Asp Ser Leu Ser Glu Phe Leu
   1 5 10
<210> 34
   <211> 9
   <212> PRT
   <213> Homo sapiens
<400> 34
   Gln Gln Tyr Lys Gly Tyr Pro Leu Thr
   1 5
<210> 35
   <211> 16
   <212> PRT
   <213> Homo sapiens
<400> 35
   Glu Ile Arg Ala Pro Asp His His Asp Leu Ser Gly Tyr Leu Gly Arg
   1 5 10 15
<210> 36
   <211> 16
   <212> PRT
   <213> Homo sapiens
<400> 36
   Glu Ile Arg Ala Pro Asn His His Asp Phe Ser Gly Tyr Leu Gly Arg
   1 5 10 15
<210> 37
   <211> 16
   <212> PRT
   <213> Homo sapiens
<400> 37
   Glu Val Arg Ala Pro Asp His His Asp Phe Ser Gly Tyr Leu Gly Arg
   1 5 10 15
<210> 38
   <211> 16
   <212> PRT
   <213> Homo sapiens
<400> 38
   Glu Ile Arg Ala Pro Asp His His Asp Phe Ser Gly Tyr Leu Gly Cys
   1 5 10 15
<210> 39
   <211> 9
   <212> PRT
   <213> Homo sapiens
<400> 39
   Asp Val Ser Ala Gly Gly Leu Asp Val
   1 5
<210> 40
   <211> 9
   <212> PRT
   <213> Homo sapiens
<400> 40
   Glu Ile Ser Ala Gly Ala Leu Asp Val
   1 5
<210> 41
   <211> 16
   <212> PRT
   <213> Homo sapiens
<400> 41
   Asp Val Gly Ser Gly Ser Tyr Tyr Thr Gly Gly His Trp Phe Asp Pro
   1 5 10 15
<210> 42
   <211> 16
   <212> PRT
   <213> Homo sapiens
<400> 42
   Asn Val Gly Ser Gly Ser Tyr Tyr Thr Gly Gly His Trp Phe Glu Pro
   1 5 10 15
<210> 43
   <211> 16
   <212> PRT
   <213> Homo sapiens
<400> 43
   Asn Val Gly Ser Gly Ser Tyr Tyr Thr Gly Gly His Trp Leu Asp Pro
   1 5 10 15
<210> 44
   <211> 16
   <212> PRT
   <213> Homo sapiens
<400> 44
   Asn Val Gly Ser Gly Ser Tyr Tyr Thr Gly Gly His Trp Phe Asp Thr
   1 5 10 15
<210> 45
   <211> 16
   <212> PRT
   <213> Homo sapiens
<400> 45
   Asn Val Gly Ser Gly Ser Tyr Tyr Thr Gly Gly His Trp Phe Gly Pro
   1 5 10 15
<210> 46
   <211> 15
   <212> PRT
   <213> Homo sapiens
<400> 46
   Asn Val Gly Ser Gly Ser Tyr Tyr Thr Ala Thr Cys Phe Asp Pro
   1 5 10 15
<210> 47
   <211> 17
   <212> PRT
   <213> Homo sapiens
<400> 47
   Gly Ala Lys His Phe Glu Gly Ser Gly Ser Trp Phe Ser Trp Phe Asp
   1 5 10 15
   Pro
<210> 48
   <211> 11
   <212> PRT
   <213> Homo sapiens
<400> 48
   Asn Ser Arg Asp Asn Thr Gly His His Val Val
   1 5 10
<210> 49
   <211> 11
   <212> PRT
   <213> Homo sapiens
<400> 49
   Asn Ser Arg Asp Ser Ser Gly Asn His Val Val
   1 5 10
<210> 50
   <211> 11
   <212> PRT
   <213> Homo sapiens
<400> 50
   Ser Ser Arg Asp Asn Ser Gly Asp Arg Tyr Val
   1 5 10
<210> 51
   <211> 10
   <212> PRT
   <213> Homo sapiens
<400> 51
   Asn Ser Arg Asp Gly Thr Gly Asn Gln Leu
   1 5 10
<210> 52
   <211> 10
   <212> PRT
   <213> Homo sapiens
<400> 52
   Asn Ser Arg Asp Thr Asn Gly Asp Gln Leu
   1 5 10
<210> 53
   <211> 11
   <212> PRT
   <213> Homo sapiens
<400> 53
   Asn Ser Arg Asp Ser Ser Gly Tyr His Val Ile
   1 5 10
<210> 54
   <211> 11
   <212> PRT
   <213> Homo sapiens
<400> 54
   Ser Ser Arg Asp Ser Lys Gly His Arg Tyr Val
   1 5 10
<210> 55
   <211> 11
   <212> PRT
   <213> Homo sapiens
<400> 55
   Ser Ser Arg Asp Ser Asn Gly Asn Arg Tyr Val
   1 5 10
<210> 56
   <211> 11
   <212> PRT
   <213> Homo sapiens
<400> 56
   Ser Ser Arg Asp Thr Lys Gly His Arg Tyr Val
   1 5 10
<210> 57
   <211> 10
   <212> PRT
   <213> Homo sapiens
<400> 57
   Lys Pro Arg Asp Ser Ser Gly Asn His Val
   1 5 10
<210> 58
   <211> 11
   <212> PRT
   <213> Homo sapiens
<400> 58
   Thr Ser Arg Asp Ser Asn Gly Asn Arg Tyr Val
   1 5 10
<210> 59
   <211> 11
   <212> PRT
   <213> Homo sapiens
<400> 59
   Ala Ala Trp Asp Asp Asn Leu Ser Ala Tyr Val
   1 5 10
<210> 60
   <211> 12
   <212> PRT
   <213> Homo sapiens
<400> 60
   Ala Ala Trp Asp Asp Ser Leu Gly Gly Lys Tyr Val
   1 5 10
<210> 61
   <211> 11
   <212> PRT
   <213> Homo sapiens
<400> 61
   Ala Ala Trp Asp Asp Ser Leu Asn Gly Val Val
   1 5 10
<210> 62
   <211> 11
   <212> PRT
   <213> Homo sapiens
<400> 62
   Ala Ala Trp Asp Asp Ser Val Lys Gly Trp Val
   1 5 10
<210> 63
   <211> 11
   <212> PRT
   <213> Homo sapiens
<400> 63
   Ala Ala Trp Asp Asp Ser Val Arg Gly Trp Val
   1 5 10
<210> 64
   <211> 11
   <212> PRT
   <213> Homo sapiens
<400> 64
   Ala Ala Trp Asp Asp Ser Val Arg Ser Trp Val
   1 5 10
<210> 65
   <211> 11
   <212> PRT
   <213> Homo sapiens
<400> 65
   Ala Ser Trp Asp Asp Ser Leu Asn Gly Val Val
   1 5 10
<210> 66
   <211> 11
   <212> PRT
   <213> Homo sapiens
<400> 66
   Ala Ser Trp Asp Asp Ser Gln Ala Ala Leu Val
   1 5 10
<210> 67
   <211> 11
   <212> PRT
   <213> Homo sapiens
<400> 67
   Ser Ala Trp Asp Ser Ser Leu Ser Thr Trp Val
   1 5 10
<210> 68
   <211> 11
   <212> PRT
   <213> Homo sapiens
<400> 68
   Gln Ser Tyr Asp Ser Ser Leu Ser Gly Trp Val
   1 5 10
<210> 69
   <211> 10
   <212> PRT
   <213> Homo sapiens
<400> 69
   Ser Ser Tyr Thr Asn Thr Asn Pro Tyr Val
   1 5 10
<210> 70
   <211> 9
   <212> PRT
   <213> Homo sapiens
<400> 70
   Gln Gln Phe Lys Ser Phe Pro Leu Thr
   1 5
<210> 71
   <211> 9
   <212> PRT
   <213> Homo sapiens
<400> 71
   Gln Gln Phe Gln Ser Tyr Pro Val Thr
   1 5
<210> 72
   <211> 9
   <212> PRT
   <213> Homo sapiens
<400> 72
   Gln Gln Tyr Lys Ser Tyr Pro Leu Thr
   1 5
<210> 73
   <211> 9
   <212> PRT
   <213> Homo sapiens
<400> 73
   His Gln Tyr Tyr Arg Phe Pro Leu Thr
   1 5
<210> 74
   <211> 9
   <212> PRT
   <213> Homo sapiens
<400> 74
   Leu Gln Tyr Lys Lys Trp Pro Leu Thr
   1 5
<210> 75
   <211> 9
   <212> PRT
   <213> Homo sapiens
<400> 75
   Gln His Phe Ser His Tyr Pro Leu Thr
   1 5
<210> 76
   <211> 9
   <212> PRT
   <213> Homo sapiens
<400> 76
   Gln His Tyr Tyr Ser Tyr Pro Leu Thr
   1 5
<210> 77
   <211> 18
   <212> PRT
   <213> Homo sapiens
<400> 77
   Asp Gly Val Arg Gln Tyr Asn Gly Gly Arg Tyr Ser Asn His Gly Met
   1 5 10 15
   Asp Val
<210> 78
   <211> 18
   <212> PRT
   <213> Homo sapiens
<400> 78
   Asp Gly Val Arg Gln Tyr Ser Gly Gly Lys Tyr Ser Asn His Gly Met
   1 5 10 15
   Asp Val
<210> 79
   <211> 18
   <212> PRT
   <213> Homo sapiens
<400> 79
   Asp Gly Val Arg Gln Tyr Ser Gly Gly Arg Tyr Ser Asn His Gly Met
   1 5 10 15
   Asn Val
<210> 80
   <211> 18
   <212> PRT
   <213> Homo sapiens
<400> 80
   Asp Gly Val Ser Gln His Asn Gly Gly Arg Tyr Ser Asn His Gly Met
   1 5 10 15
   Asp Val
<210> 81
   <211> 18
   <212> PRT
   <213> Homo sapiens
<400> 81
   Asp Gly Val Ser Gln Tyr Ser Gly Gly Arg Tyr Ser Asn His Gly Met
   1 5 10 15
   Asp Val
<210> 82
   <211> 18
   <212> PRT
   <213> Homo sapiens
<400> 82
   Asn Gly Val Arg Gln Tyr Ser Gly Gly Arg Tyr Ser Asn His Arg Met
   1 5 10 15
   Asp Val
<210> 83
   <211> 18
   <212> PRT
   <213> Homo sapiens
<400> 83
   Glu Glu Glu Arg Gln Tyr Ser Gly Gly Arg Tyr Ser Asn His Gly Met
   1 5 10 15
   Asp Val
<210> 84
   <211> 18
   <212> PRT
   <213> Homo sapiens
<400> 84
   Asp Gly Val Arg Gln Tyr Ser Gly Ala Asp Thr Pro Asn His Gly Met
   1 5 10 15
   Asp Val
<210> 85
   <211> 10
   <212> PRT
   <213> Homo sapiens
<400> 85
   Leu Thr Ala Ala Gly Ala His Phe Asp Pro
   1 5 10
<210> 86
   <211> 10
   <212> PRT
   <213> Homo sapiens
<400> 86
   Leu Thr Ala Ala Gly Gly Arg Phe Asp Pro
   1 5 10
<210> 87
   <211> 11
   <212> PRT
   <213> Homo sapiens
<400> 87
   His Ser Arg Asp Asp Thr His Tyr Pro Val Ile
   1 5 10
<210> 88
   <211> 10
   <212> PRT
   <213> Homo sapiens
<400> 88
   Asn Ser Arg Asp Ser Asn Asn His Val Val
   1 5 10
<210> 89
   <211> 9
   <212> PRT
   <213> Homo sapiens
<400> 89
   Gln Ser Arg Asn Asn Thr Tyr Val Leu
   1 5
<210> 90
   <211> 11
   <212> PRT
   <213> Homo sapiens
<400> 90
   His Thr Arg Asp Thr Leu Phe Pro Val Asp Phe
   1 5 10
<210> 91
   <211> 19
   <212> PRT
   <213> Homo sapiens
<400> 91
   Asp Met Gly Met Phe Cys Ser Gly Val Ile Cys Tyr Asp Tyr Tyr Gly
   1 5 10 15
   Met Asp Val
<210> 92
   <211> 4
   <212> PRT
   <213> Artificial Sequence
<220>
   <223> Used for defining CDR region
<220>
   <221> MISC_FEATURE
   <222> (3)..(3)
   <223> Any amino acid
<400> 92
   Phe Gly Xaa Gly
   1
<210> 93
   <211> 4
   <212> PRT
   <213> Artificial Sequence
<220>
   <223> Used for defining CDR region
<220>
   <221> MISC_FEATURE
   <222> (2)..(4)
   <223> Any amino acid
<400> 93
   Cys Xaa Xaa Xaa
   1
<210> 94
   <211> 5
   <212> PRT
   <213> Artificial Sequence
<220>
   <223> Used for defining CDR region
<400> 94
   Leu Glu Trp Ile Gly
   1 5
<210> 95
   <211> 4
   <212> PRT
   <213> Artificial Sequence
<220>
   <223> Used for defining CDR region
<220>
   <221> MISC_FEATURE
   <222> (3)..(3)
   <223> Any amino acid
<400> 95
   Trp Gly Xaa Gly
   1
<210> 96
   <211> 10
   <212> PRT
   <213> Homo sapiens
<400> 96
   Gln Ser Tyr Asp Ser Ser Arg Gly Arg Val
   1 5 10
<210> 97
   <211> 9
   <212> PRT
   <213> Homo sapiens
<400> 97
   Met Gln Gly Ile Arg Pro Pro Arg Thr
   1               5